# EUROPEAN PATENT SPECIFICATION

(11) **EP 3 749 398 B1**
(45) Date of publication and mention of the grant of the patent: **28.01.2026**
(21) Application number: 19714888.5
(22) Date of filing: 07.02.2019
(51) Int. Cl.: A61M 5/32

(54) **SAFETY NEEDLE DEVICE**
SICHERHEITSNADELVORRICHTUNG
DISPOSITIF D'AIGUILLE DE SÉCURITÉ

(30) Priority: 08.02.2018 PL 42454518
(43) Date of publication of application: 16.12.2020
(73) Proprietor: HTL-Strefa Spolka Akcyjna, 95-035 Ozorkow (PL)
(72) Inventor: KOMUDA, Marcin, 01-163 Warszawa (PL); ROZWADOWSKI, Marcin, 01-864 Warszawa (PL); NIEMIEC, Marcin, 05-500 Piaseczno (PL); GRZELAK, Robert, 03-042 Warszawa (PL); KARBOWNICZEK, Jacek, 05-080 Lipków (PL)
(74) Representative: Perani & Partners S.p.A.
(86) International application number: PCT/PL2019/050010
(87) International publication number: WO 2019/156579

(56) References cited:
- EP-A1- 2 918 301
- WO-A1-93/01851
- US-A1- 2013 204 186

## Description

The subject of the invention is a safety needle device inserted with first end into the patient's body and connected with second end to a supply vessel for a substance administered to the patient or a recipient vessel for a sample of the patient's tissue, in particular a body fluid, which prevents repeated use of the same needle and a needlestick injury with a used needle.

Safety needle devices are known and used in medicine and include an elongated needle with an in-axis channel, with first end inserted into the patient's body and embedded in a hub of the device in the vicinity of the second end of the needle. The hub of the device is equipped with means for connecting the needle channel with a vessel for supplying a substance to be injected or a vessel for a patient's tissue sample. Known devices also have a housing fixed on the said hub and surrounding the needle along a part of its length, and a hollow slider surrounding the needle and moving freely along the needle's axis. The slider has first end directed towards the outside of the housing and a second end moving inside the housing and directed towards the hub, as well as means preventing the slider from rotating relative to the housing and the second end of the slider from sliding out of the housing. Such device also includes a longitudinal resilient means, e.g. a spring, placed inside the housing, with its first end backed against the slider and its second end backed against the hub. The spring is at its lowest level of tension when the slider is maximally extended with its first end from the housing and the spring tension increases as the needle is inserted deeper, with the slider being simultaneously inserted deeper into the housing. During the needle withdrawal from the patient's body, the tensioned spring pushes the slider out of the housing but the slider still covers the visible part of the needle. The length of the slider is chosen so that when it is fully extended from the housing, the first end of the needle is hidden inside. Since the device also includes means to block slider movements when the slider reaches the position most extended out of the housing once the needle is removed from the patient's body after insertion, re-insertion or even accidental needle-stick injury by careless handling is impossible. Embodiments of a safety needle device are disclosed in patents WO2015/047114 and US5549558 Further embodiments of a safety needle device are disclosed in WO 9301851 A1.

The goal of the invention was to develop a safety needle device design that activates automatically, i.e. does not require any additional user actions. This goal is achieved by a device according to the invention, comprising:
- an elongated needle with an in-axis channel, with its first end inserted into the patient's body and embedded in a hub in its second end area, wherein the hub is provided with means for connecting the needle channel with a vessel for supplying an injectable substance or a vessel for receiving a sample of the patient's tissue;
- a housing fixed immovably on the hub and surrounding the needle along a part of its length;
- a hollow slider surrounding the needle and moving freely along the needle's axis, having first end directed towards the outside of the housing and second end moving inside the housing and directed towards the hub, as well as means preventing the slider from rotating relative to the housing and from sliding the second end of the slider out of the housing;
- a longitudinal resilient means located inside the housing, with its first end backed against the slider and with its second end against the body, with it at the lowest level of tension when the slider is most extended with its first end from the housing;
- means for blocking the slider's movement in the position most extended out of the housing after the first end of the needle is removed from the patient's body. The invention consists in that:
- in the device housing there is a cylindrical channel with an axis parallel to the needle's axis;
- in the cylindrical channel of this housing there is a rotary sleeve surrounding the needle, a longitudinal resilient means and at least the second end of the slider;
- in a side wall of the rotary sleeve there is at least one linear notch with its axis along the needle's axis; the linear notch has a first and a second end, and a start bay and a blocking bay; the first end of the linear notch is closer to the first end of the needle than the second end of the linear notch;
- in the vicinity of the second end of the slider there is at least one pin located transversely to the needle's axis and coupled mechanically to the linear notch in the side wall of the rotating sleeve.

In one variant of the invention, the device's hub has, on its circumference, means for connecting the said hub to the housing. The hub has an axial central member, in which the second end of the needle is seated, and a retaining element for the second end of the longitudinal resilient means surrounding the central member.

In another variant of the invention, the longitudinal resilient means is a compression helical spring coaxial in relation to the needle and surrounding the central member.

In next variant of the invention, the start bay of the linear notch of the rotary sleeve is located at first end of the notch and is offset against the axis of the linear notch.

In next variant of the invention, the blocking bay of the linear notch of the rotary sleeve is located in the immediate vicinity of the start bay. Both bays together with the linear notch form a notch in the side wall of the rotary sleeve with a shape similar to the letter "Y".

In next variant of the invention, the side wall of the rotary sleeve has two opposing linear notches with bays, and the slider has two opposing pins.

In next variant of the invention, the blocking bay of the linear notch of the rotary sleeve is located at its first end and is offset against the axis of the linear notch. The start bay is located between the first and second end of the linear notch.

In next variant of the invention, the side wall of the rotary sleeve has two opposite linear notches. Each of these two notches has two bays and the device's slider has two opposing pins.

In next variant of the invention, the device's slider has at least one longitudinal guide on the outer surface of the said slider. The device housing has at least one guide notch corresponding to the longitudinal slider guide.

In next variant of the invention, the device's slider has two opposing longitudinal guides and the device's housing has two opposite guide notches corresponding to the longitudinal guides of the slider.

In next variant of the invention, the central member of the device's hub has at least one longitudinal guide. The device's slider has, at its second end, at least one guide notch corresponding to the longitudinal guide of the central member of the hub.

In next variant of the invention, the central member of the hub has four longitudinal guides spaced at regular intervals and the slider has four guide notches corresponding to the longitudinal guides of the said central member.

In next variant of the invention, the device includes means for indicating that the slider is blocked in position most extended from the housing.

In next variant of the invention, the means for indicating that the slider is blocked in the position most extended from the housing consist of at least one window in the side wall of the housing and at least one mark on the outer surface of the rotary sleeve.

In next variant of the invention, in the hollow part of the slider there is a narrowed section supporting the needle.

In next variant of the invention, the device's slider is made of a transparent material.

In another variant of the invention, the second end of the needle protrudes from the hub on the side opposite to the first end of the needle.

In yet another variant of the invention, the device includes a stabiliser for the second end of the needle surrounding the said needle and placed axially in the hub.

The design of the device according to the invention retains the full functionality of known devices, while demonstrating new advantages. The device operates smoothly and noiselessly during needle insertion, and the additional force required from the user in comparison with a standard needle is insignificant and increases linearly during insertion of the needle. In embodiments with a transparent slider, the user can easily assess flow, remove air from the needle and always control the position of the needle visually. The low force required to puncture the skin and the elimination of rotational movements, in particular the rotation of the slider remaining in contact with the patient's skin in known devices, substantially increase the comfort of the patient receiving the puncture. In the case of rough skin, the risk of disruptions to work known from using safety needles with a rotary slider was also eliminated. The simplicity of the blocking mechanism with the rotary sleeve has a very beneficial influence on the reliability of operations. Despite the small number of parts, versatility is much greater than before. The desired slider movement range, which influences, among other aspects, the precision of needle insertion, can be assured in a simple manner by mounting a dedicated rotary sleeve in the device, even without changing the other parts constituting the device. Similarly, a dedicated slider can easily be used in the device without modifications to other components. The relatively large diameter of the slider occurring in, for example, the first exemplary embodiment, spreads the force necessary to deflect the internal resilient means over a relatively large area of the patient's body, which minimises discomfort and leaves no signs of pressure on the skin. Simultaneously, the internal narrow section of the slider increases precision of both needle insertion and guiding. Aside from the slider, no other movable part is available to the user who cannot observe the operation of the internal mechanism or manipulate it themselves in any phase of operating the device. The operation of the slider blocking mechanism is initiated automatically and irreversibly at the beginning of the insertion, in some variants even before the needle touches the patient's body. This renders repeated use of the needle impossible, effectively protecting patients from infection, while the status of the slider block is clearly indicated. The inner spring is slightly tensioned in the initial state and a its stronger tension occurs only during use and lasts a short time. Thanks to this, the device according to the invention demonstrates a long guaranteed time of suitability for use.

When used with a universal "luer lock" or "luer slip" fitting, the device's design ensures that the unused volume of the fluid resulting from the dead space inside the needle is minimal and comparable to standard needles and needles with, so-called active safety systems, i.e. those requiring additional actions from the user to activate safety feature. Simultaneously, the versatility of the mechanism allows the use of another connection with a vessel for providing the substance to be injected or a vessel for receiving the sample of tissue, such as for example a thread allowing connection with insulin pen.

The invention, in four exemplary embodiments, is shown in drawings, wherein the first exemplary embodiment is illustrated in figures from Fig.1 to Fig.49, the second exemplary embodiment is shown in figures from Fig.50 to Fig.95, the third exemplary embodiment is shown in figures from Fig. 96 to Fig.145, while the fourth exemplary embodiment is shown in figures from Fig.146 to Fig.192.

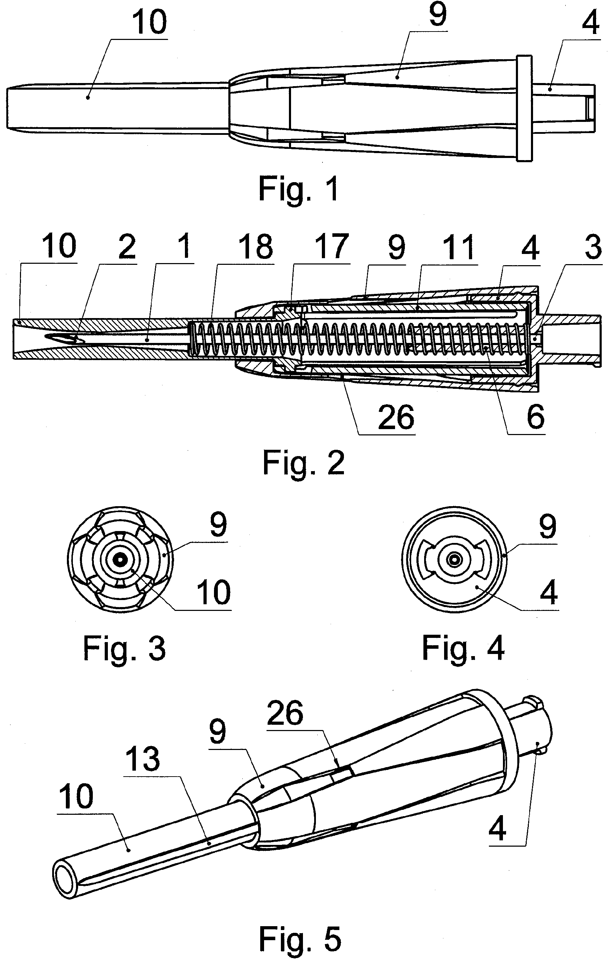

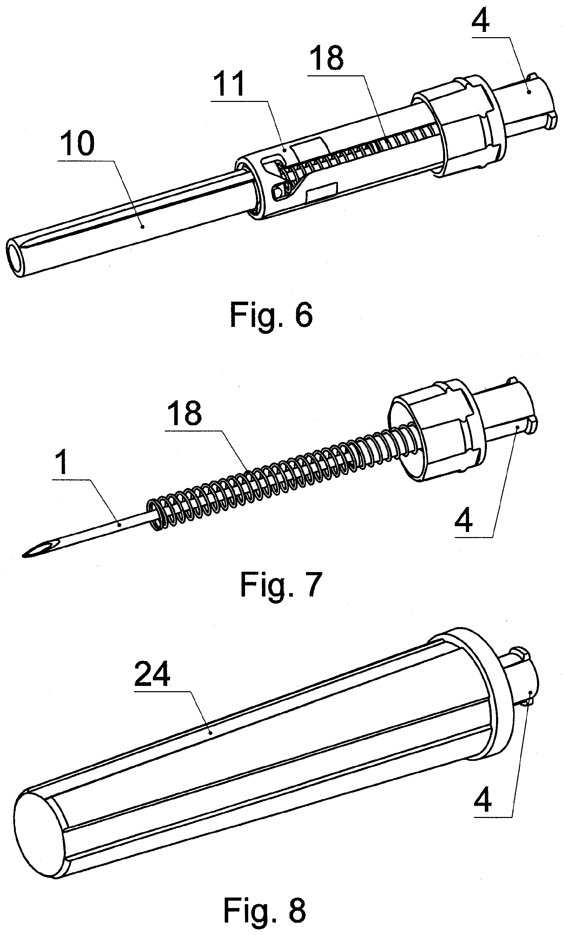

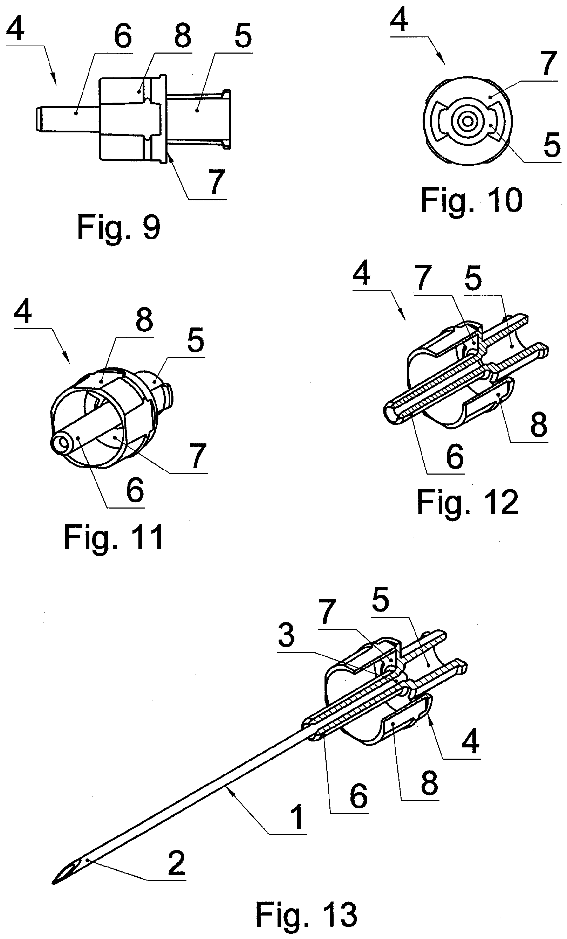

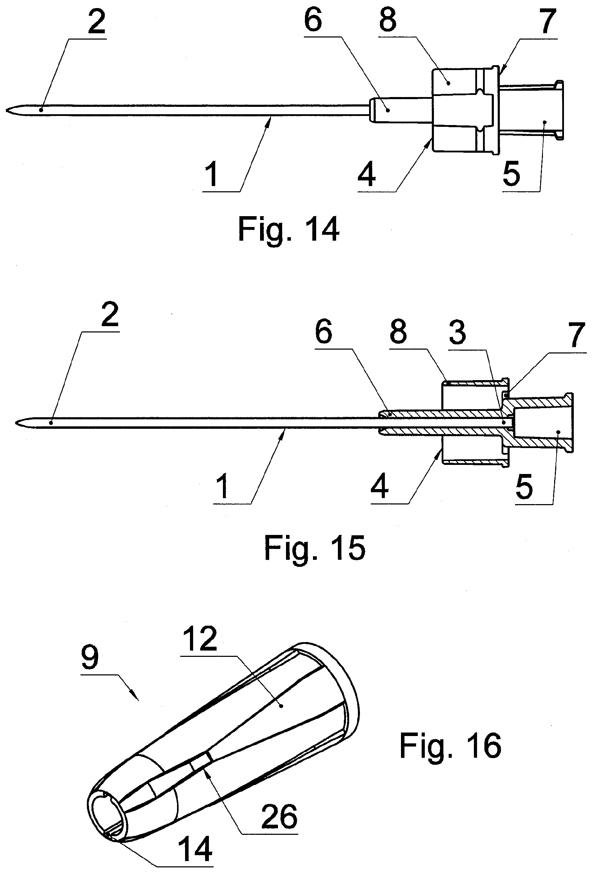

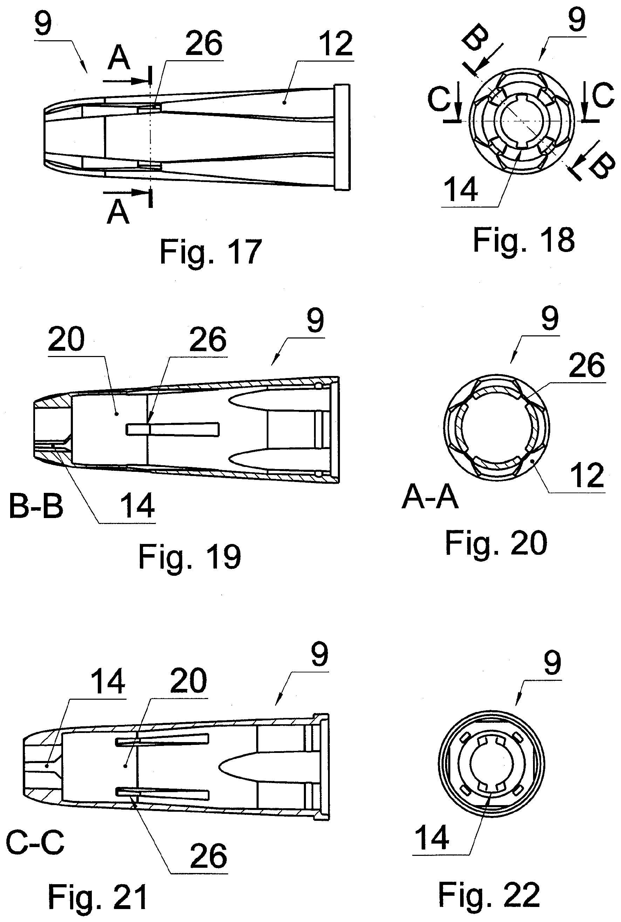

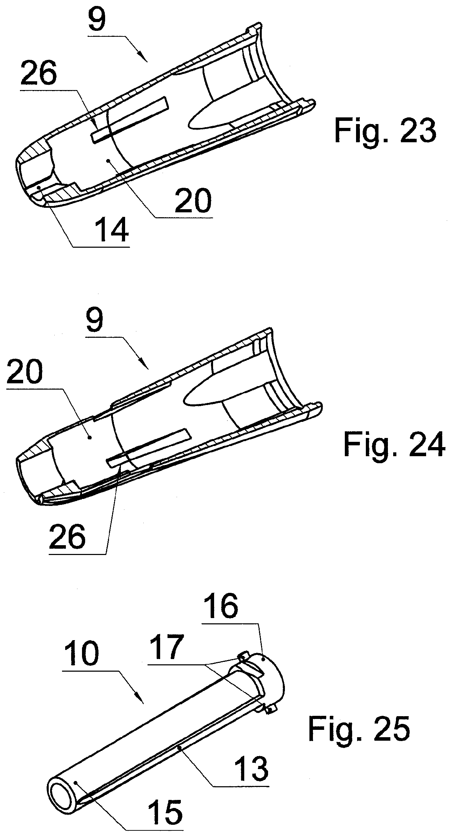

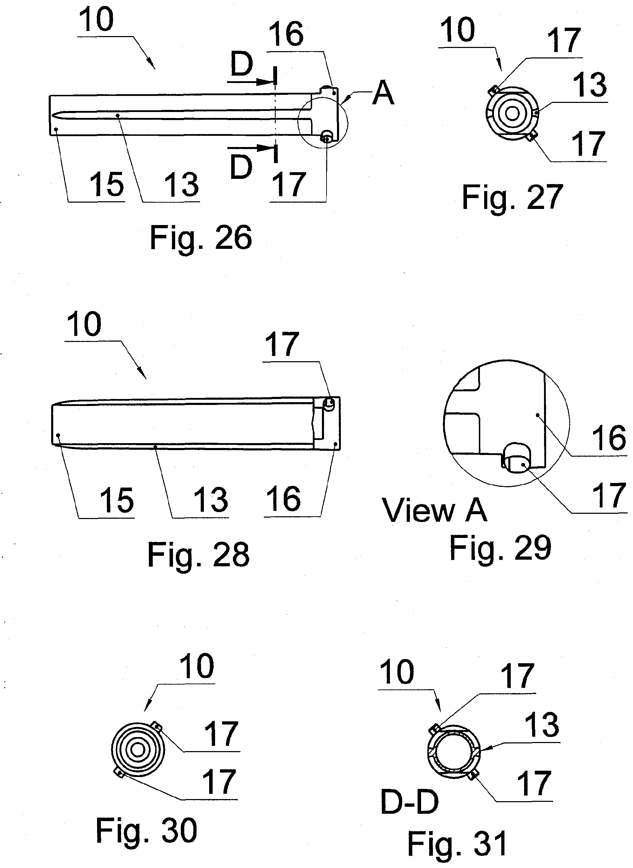

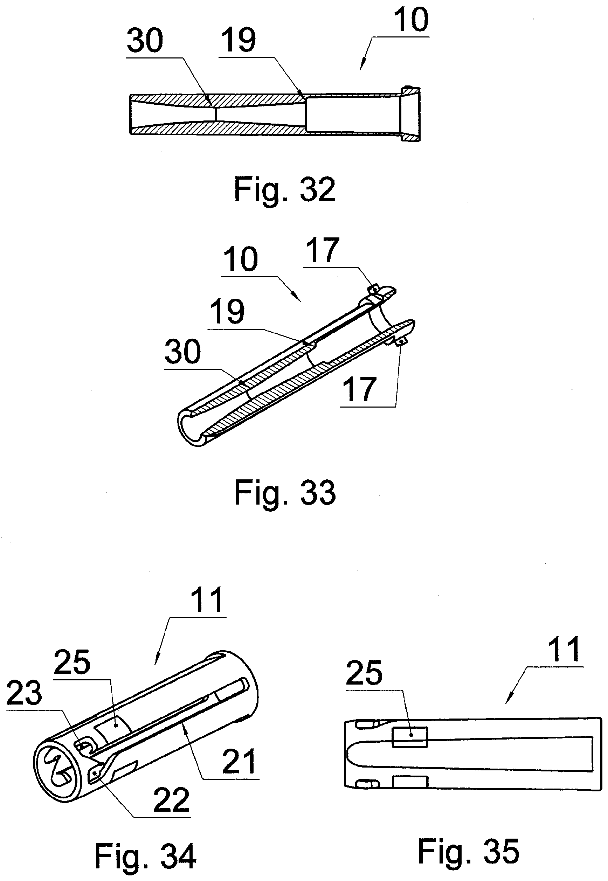

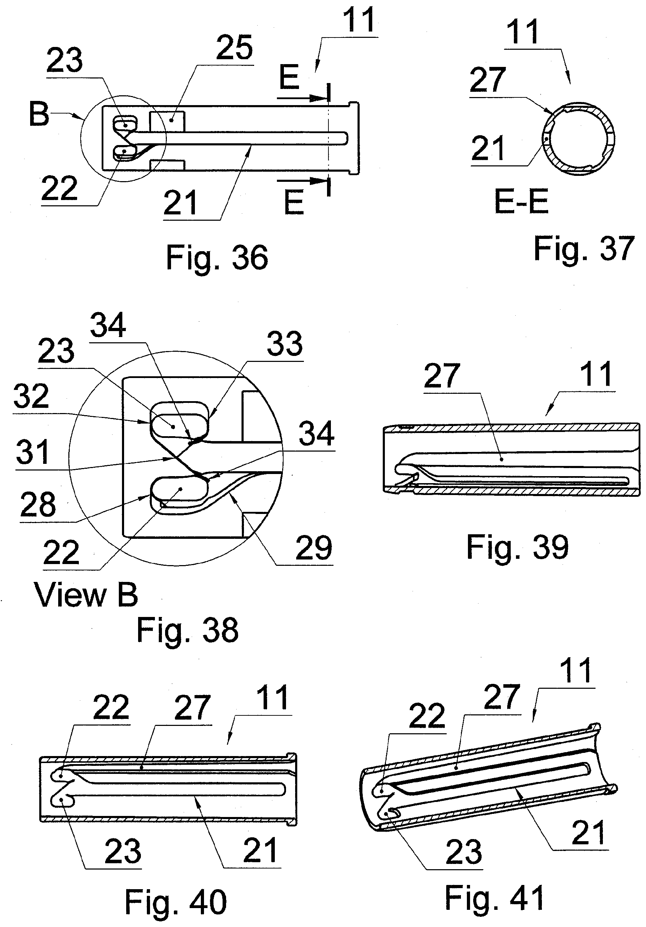

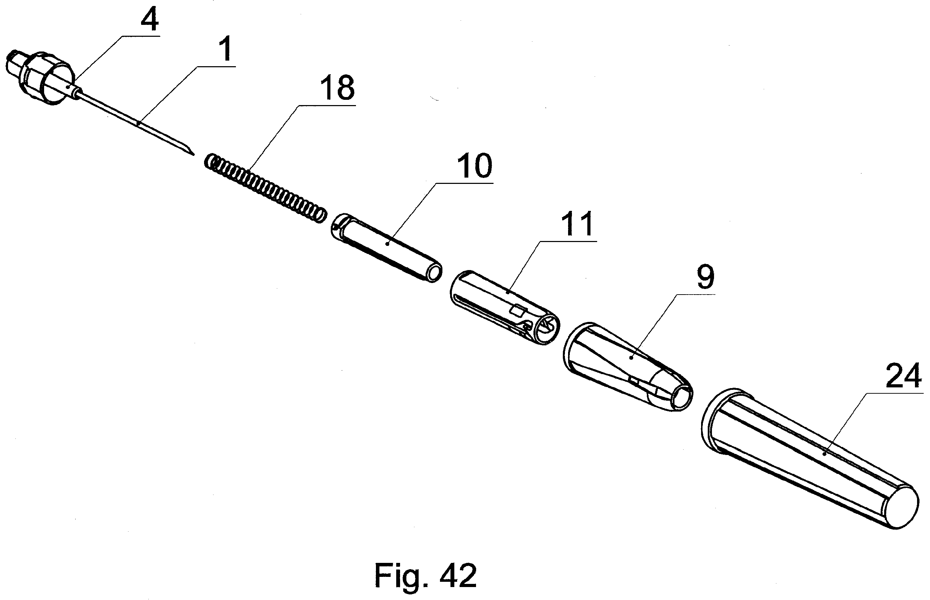

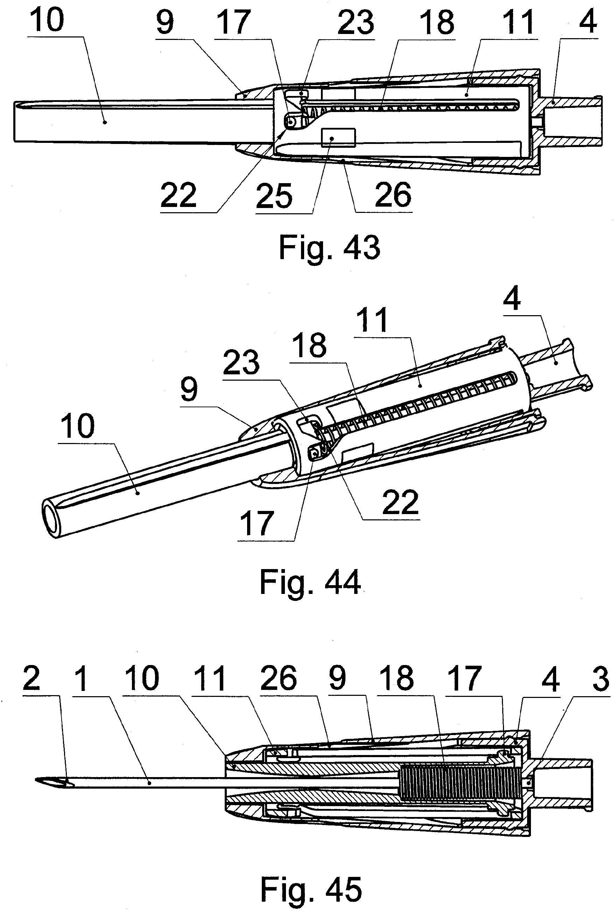

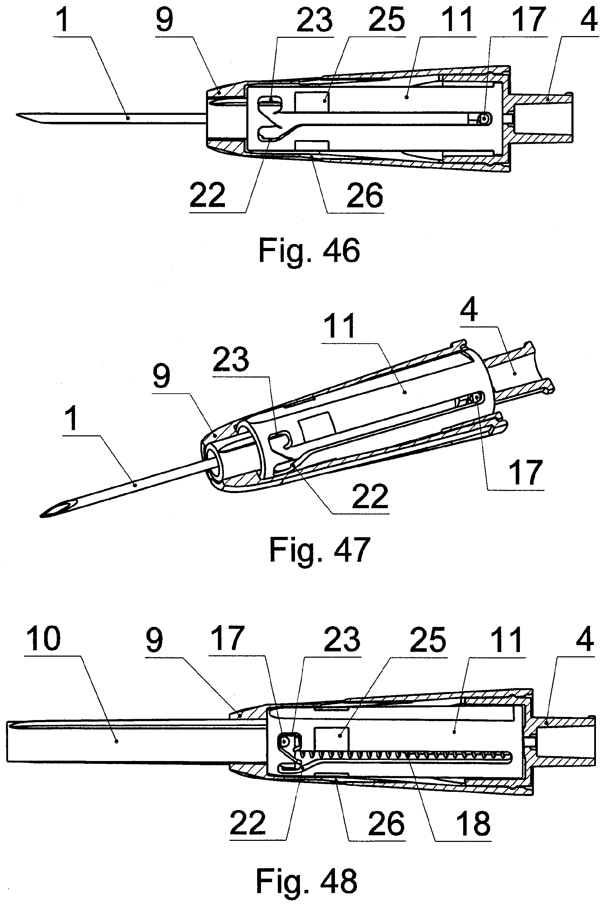

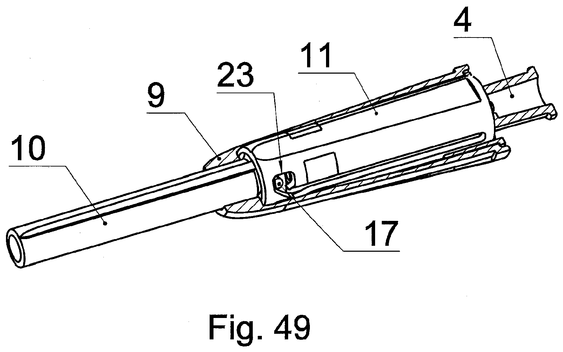

Fig.1 shows the complete device of the first exemplary embodiment in a ready-to-use state (without the cover) in a side view, while Fig.2, Fig.3, Fig.4 and Fig.5 show the same device in a longitudinal section, in a front (from the needle side) view, in rear view and in an axonometric front view, respectively. Fig.6 shows the device of Fig.1 with a housing removed. Fig.7 shows the same device after additional removal of a slider and a rotary sleeve. Fig.8 shows the device of Fig.1 with the cover applied. Figures from Fig.9 to Fig.12 show a hub of the device from Fig.1, in a side view, in a rear view, and in an axonometric front view and in the same view with cut-away, respectively. Fig.13 shows an axonometric cut-away view of the hub with cut-away and with a needle inserted therein. Fig.14 shows a side view of the hub with the needle of Fig.13 while Fig.15 shows the same view with a longitudinal section of the hub itself. Fig.16 shows an axonometric front view of the housing of the device of Fig.1. Figures from Fig.17 to Fig.22 show the housing of Fig.16 in a side view, in a front view, in a first longitudinal section, in a cross-section, in a second longitudinal section and in a rear view, respectively. Fig.23 shows an axonometric view of the second longitudinal section of the housing and Fig.24 is the same view of its first longitudinal section. Fig.25 shows an axonometric view from the front of the slider of the device from Fig 1. Figures from Fig. 26 to Fig. 33 show the slider of Fig.25 in a first side view, in a front view, in a second side view, in an enlarged fpart of the first side view, in a rear view, in a cross-section, in a longitudinal section and in a cut-out axonometric view, respectively. Fig.34 shows an axonometric view from the front of the rotary sleeve of the device of Fig.1. Figures from Fig.35 to Fig.41 show the sleeve of Fig.34 in a first side view, in a second side view, in a cross-section view, in an enlarged part of the second side view, in a first longitudinal section, in a second longitudinal section, and in an axonometric view of the second longitudinal section. Fig.42 shows the device of Fig.8 in an axonometric exploded view. Fig.43 and Fig.44 show a side view and an axonometric view of the device of Fig.1, respectively, in first phase, i.e. ready for insertion. Fig.45, Fig.46 and Fig.47 show the device of Fig.1 after insertion into the patient's body (not shown), i.e. at the end of second phase and in third phase, while Fig.48 and Fig.49 show the same device in a blocked state, i.e. in fourth phase.

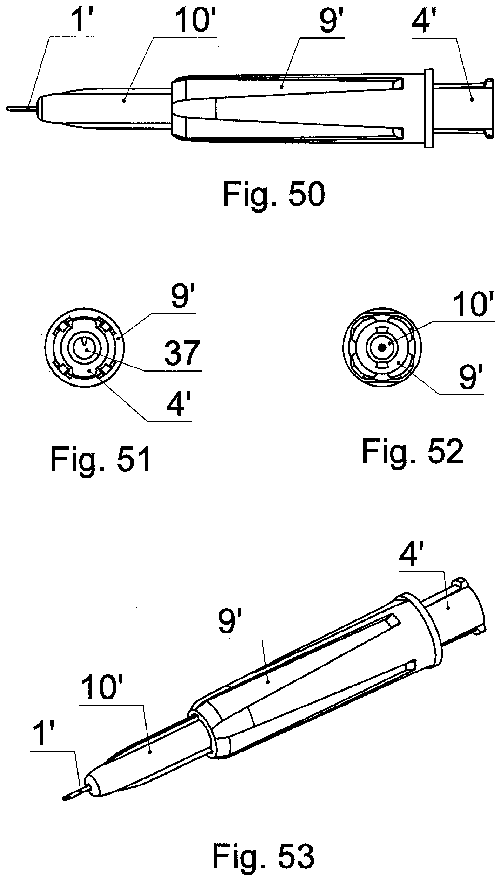

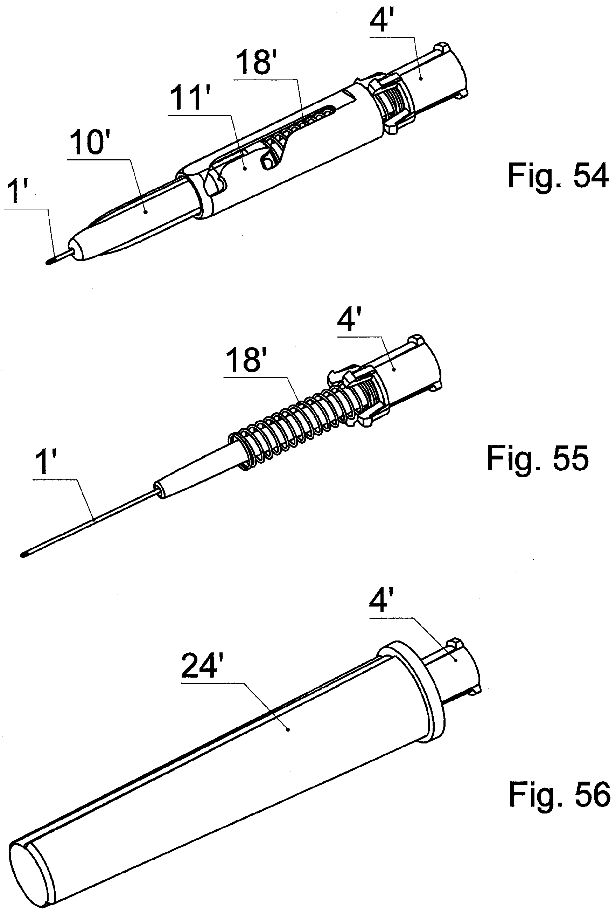

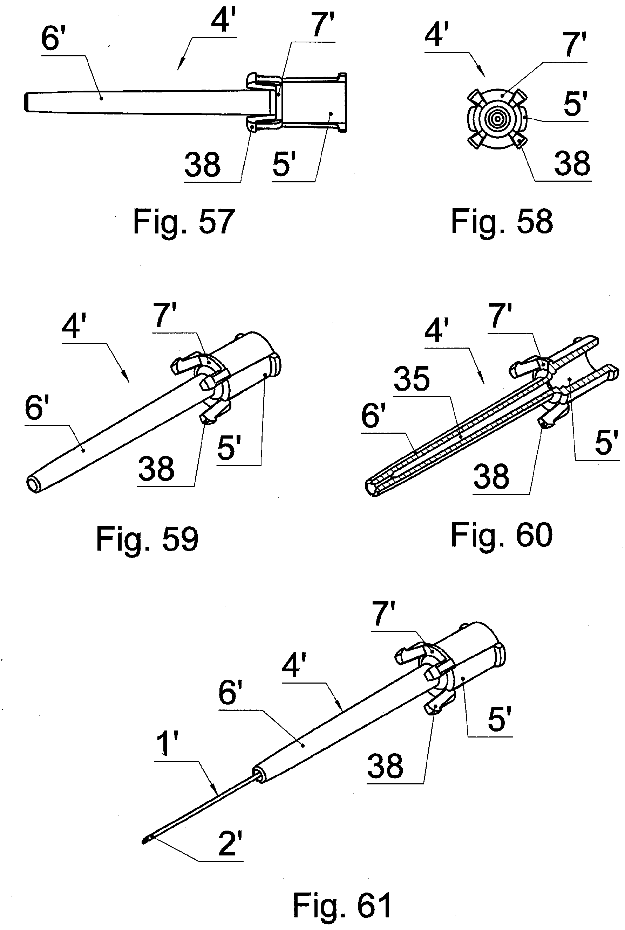

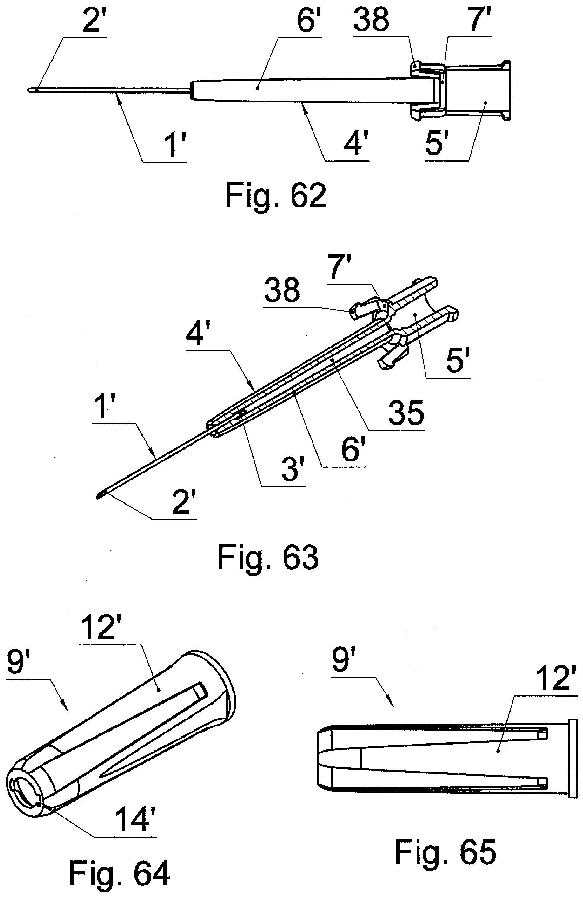

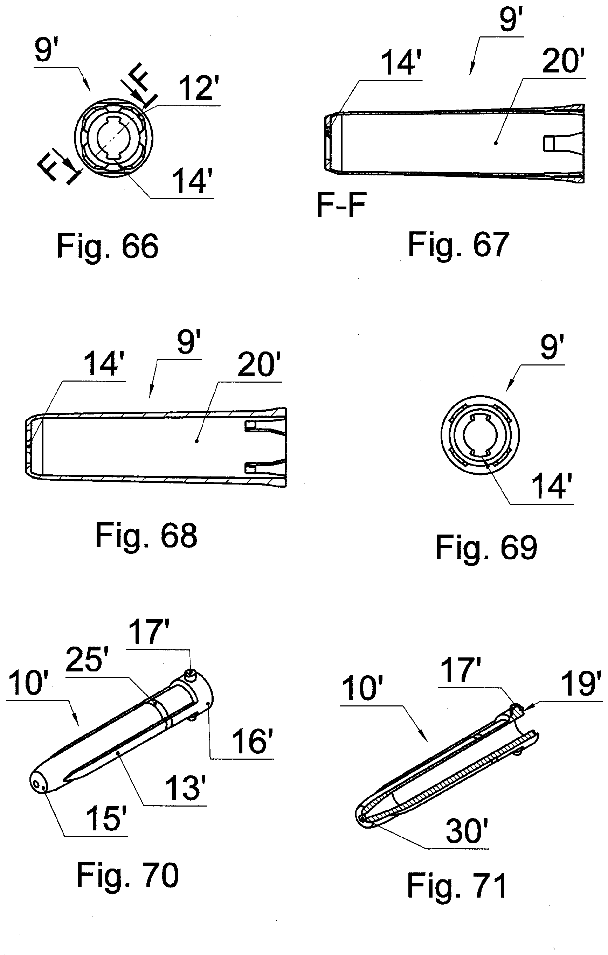

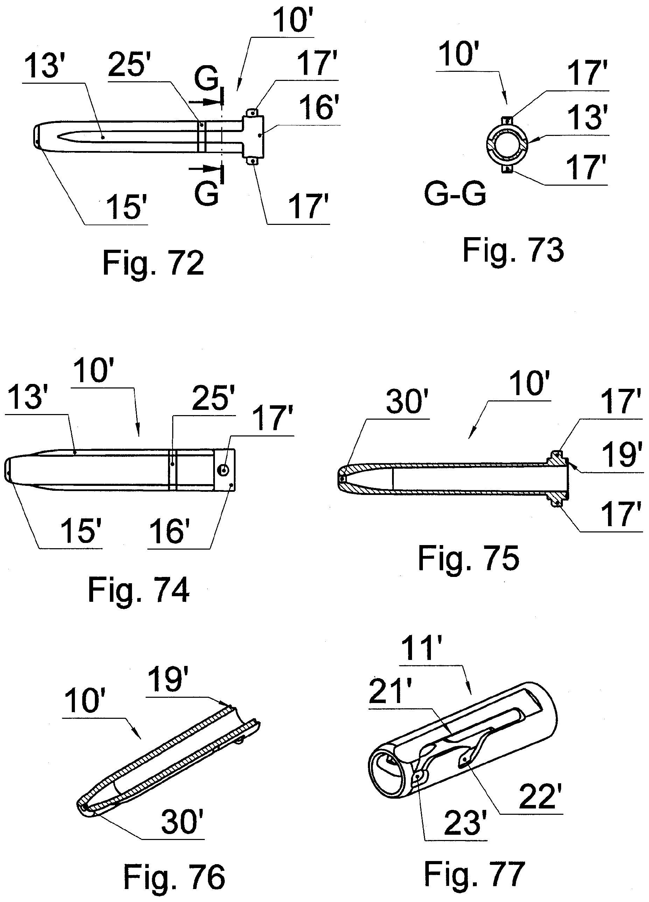

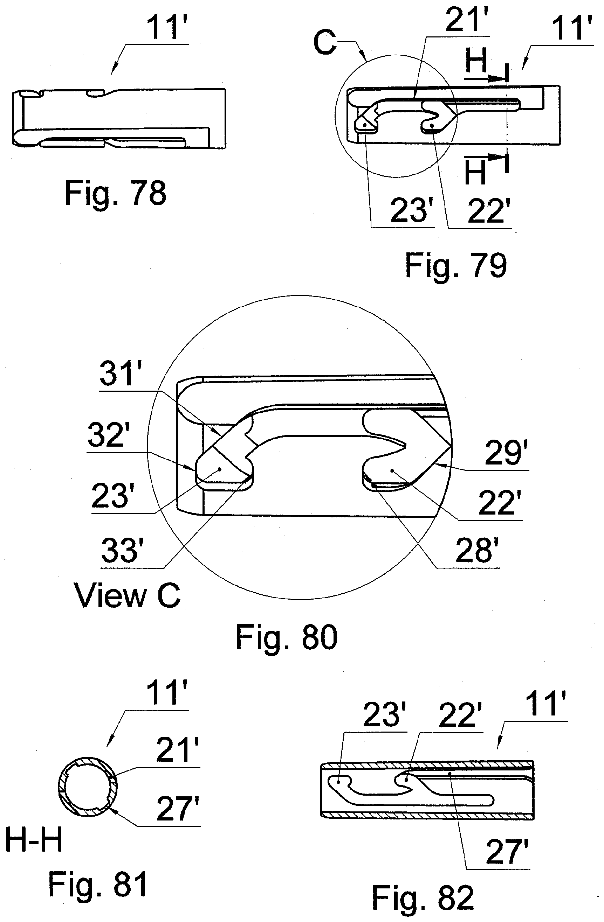

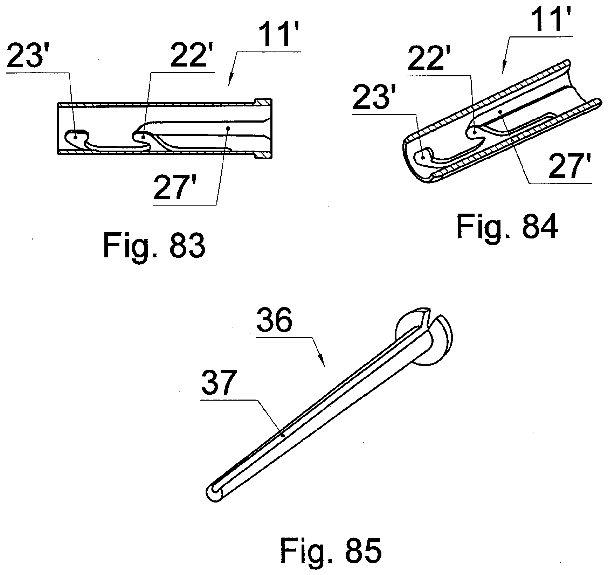

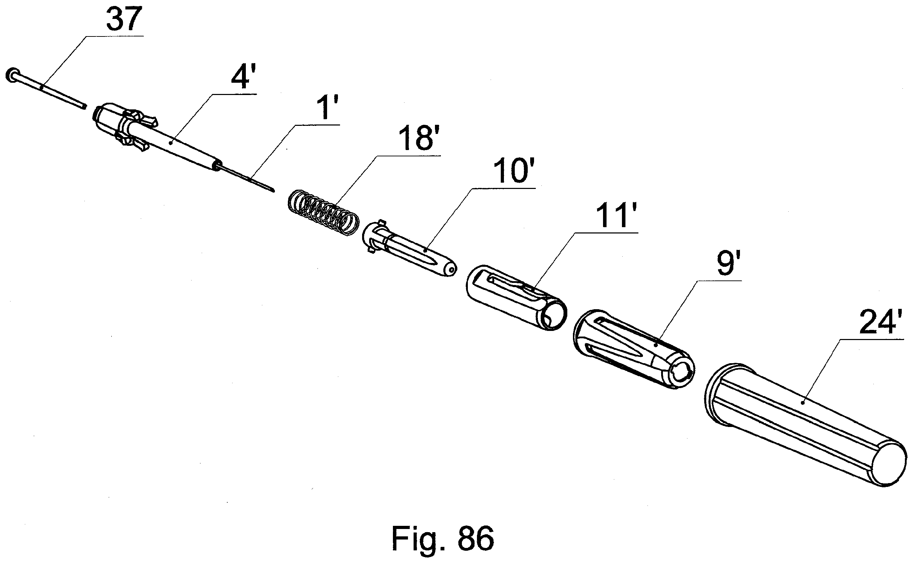

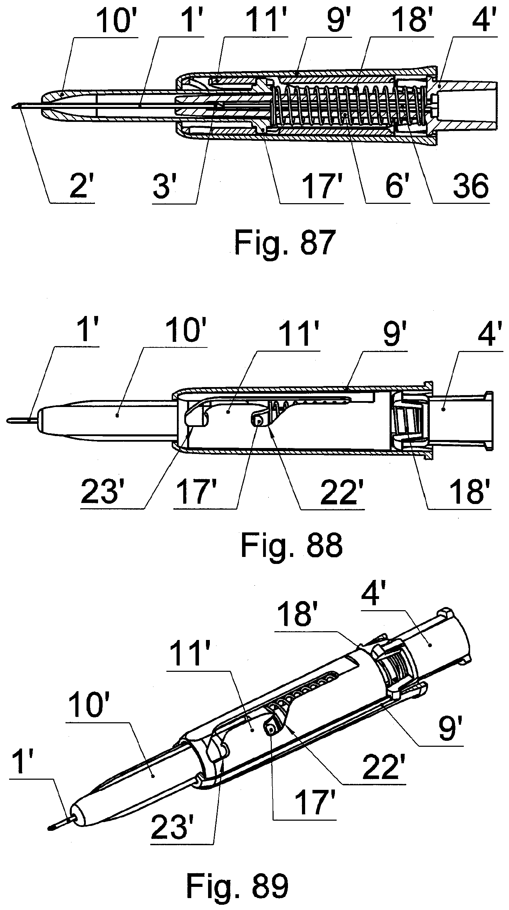

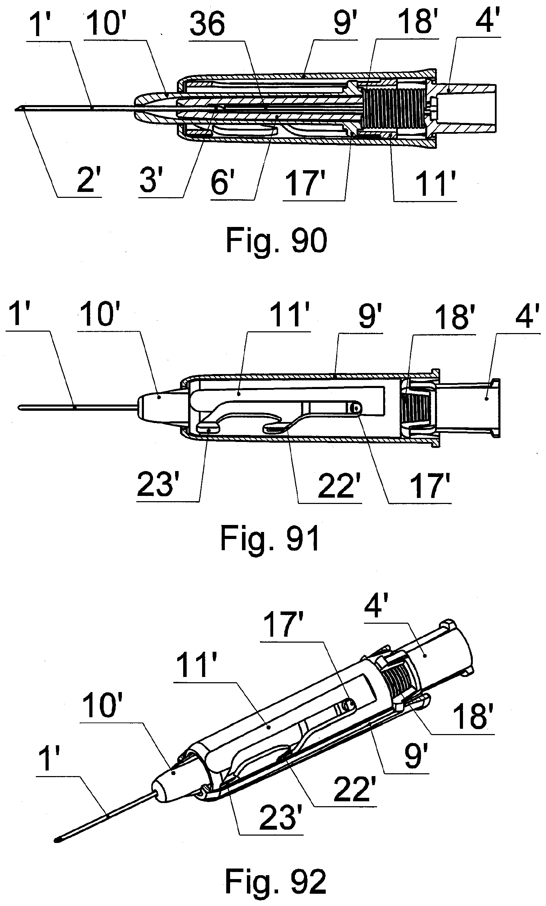

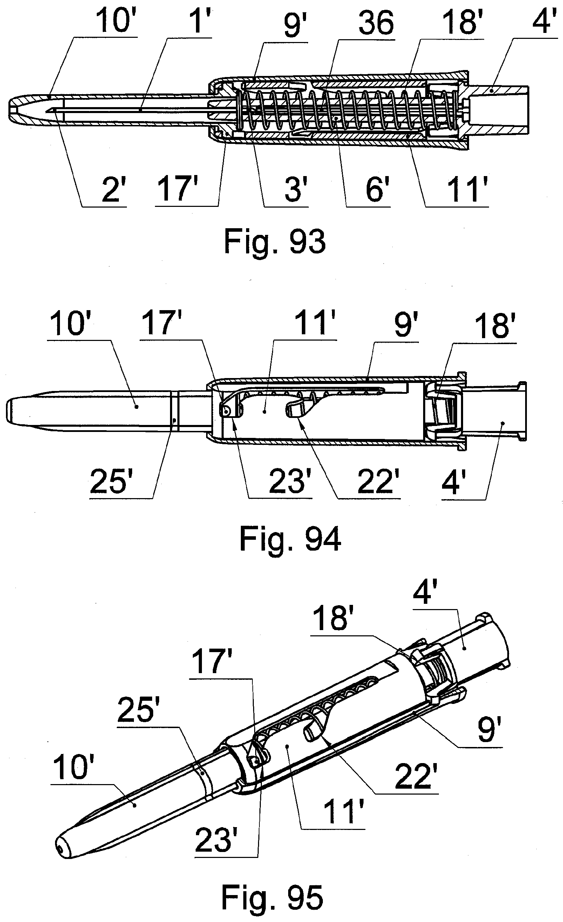

Fig. 50 shows the complete device of the second exemplary embodiment in a ready-to-use state (without the cover) in a side view, and Fig.51, Fig.52, Fig.53 show the same device in a rear, in front (from the needle side) and in an axonometric front view, respectively. Fig.54 shows the device of Fig.50 with the housing removed and Fig.55 shows the same device after additional removal of the slider and the rotary sleeve. Fig.56 shows the device of Fig. 50 with the cover applied. Figures from Fig.57 to Fig.60 show the hub of the device of Fig.50, in a side view, in front view, in axonometric view from the front and in the same view with cut-away, respectively. Fig.61 shows an axonometric view of the hub of Fig.57 with a needle placed therein, Fig.62 shows a side view of the hub with the needle, while Fig.63 shows the same hub with the needle in a cut-away axonometric view. Fig.64 shows an axonometric view from the front of the housing of the device of Fig.50. Figures from Fig.65 to Fig.69 show the housing of Fig.64 in a side view, in a front view, in a first longitudinal section, in a second longitudinal section and in a rear view, respectively. Fig.70 shows an axonometric view from the front of the slider of the device of Fig50. Figures from Fig.71 to Fig.76 show the slider of Fig.70 in an axonometric cut-away view, in a first side view, in a cross-section, in a second side view, in a longitudinal section and in an axonometric view of the longitudinal section. Fig.77 shows an axonometric view from the front of the rotary sleeve of the device from Fig. 50. Figures from Fig. 78 to Fig. 84 show the sleeve of Fig. 77 in a first side view, in a second side view, in an enlarged part of the second side view, in a cross-section, in a first longitudinal section, in a second longitudinal section and in an axonometric view of the second longitudinal section, respectively. Fig.85 shows an axonometric view of the reduction insert of the hub of Fig.57. Fig.86 shows the device of Fig.56 in an exploded view. Fig.87, Fig.88 and Fig.89 show the device of Fig.50 in first phase, i.e. ready for insertion. Fig.90, Fig.91 and Fig.92 show the device of Fig.50 after insertion into the patient's body(not shown), i.e. at the end of second phase and in third phase, while Fig.93, Fig.94 and Fig.95 show the same device in a blocked state, i.e. in fourth phase.

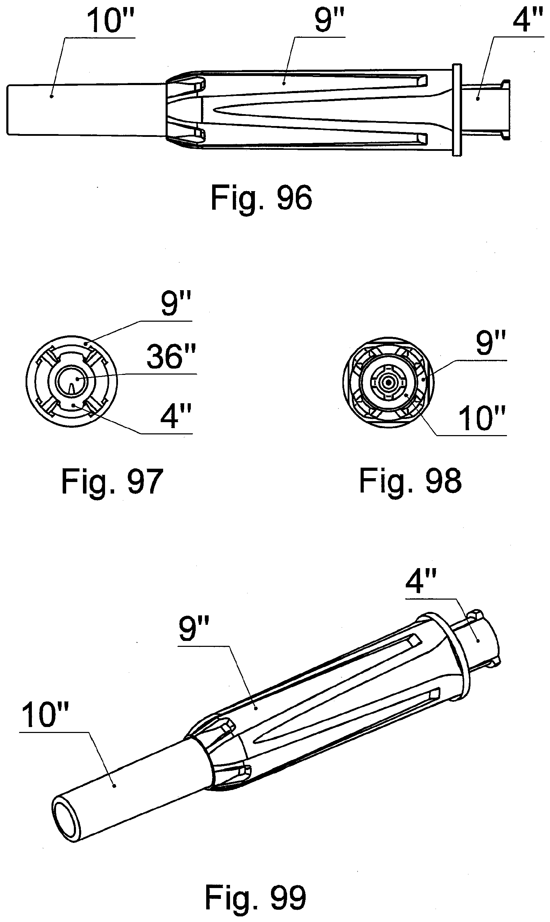

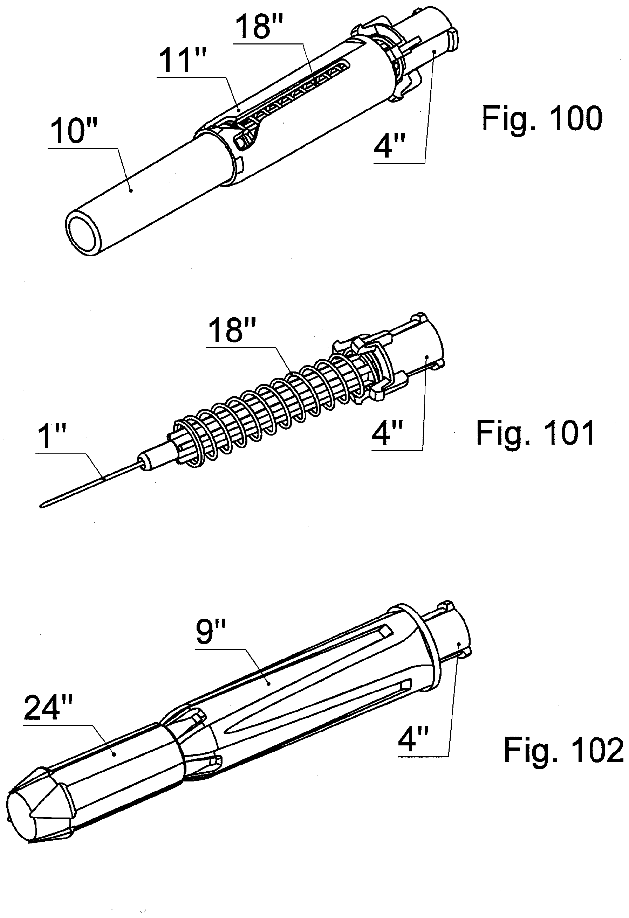

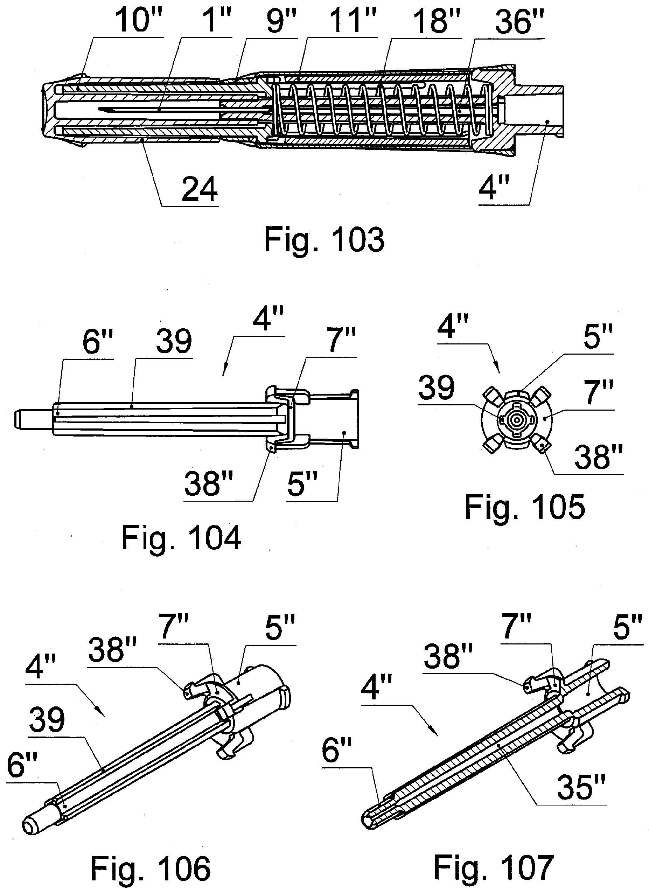

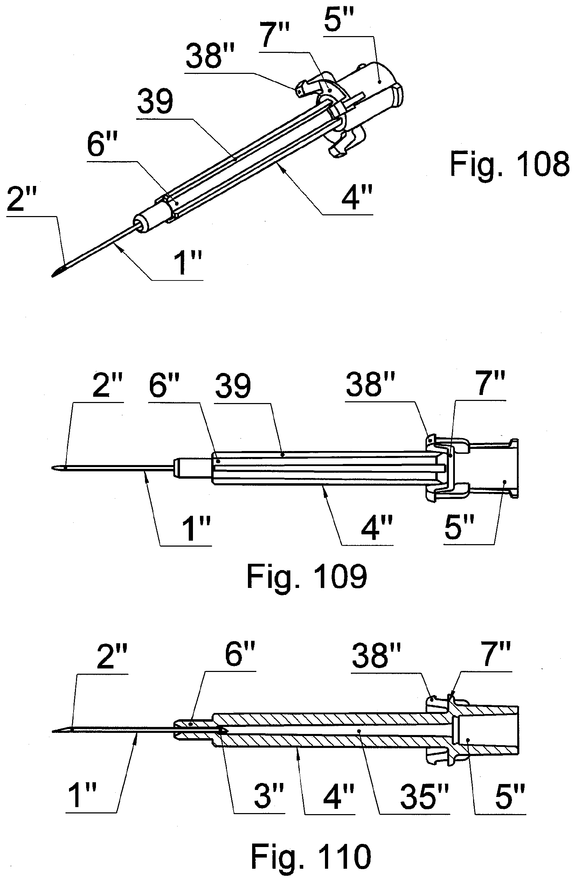

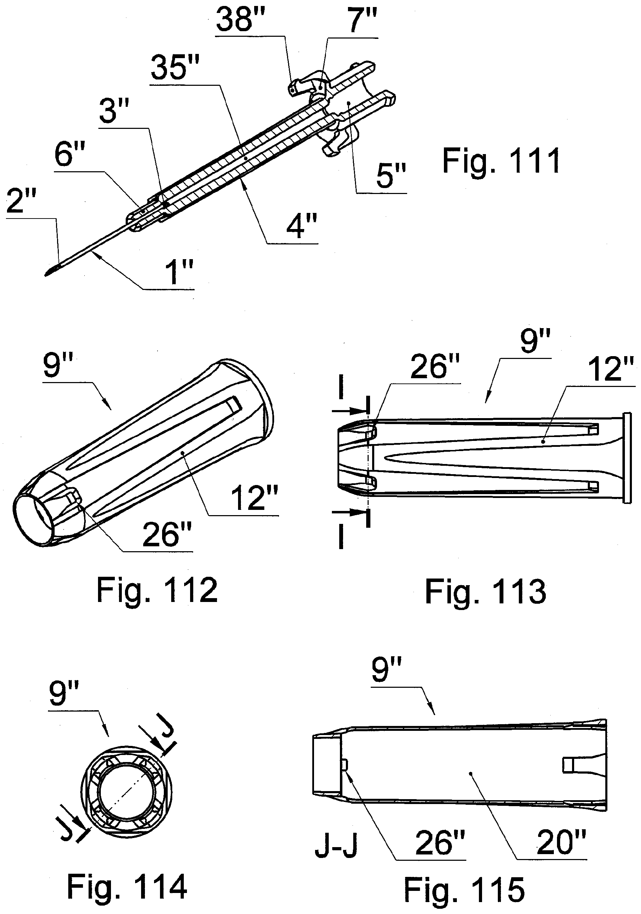

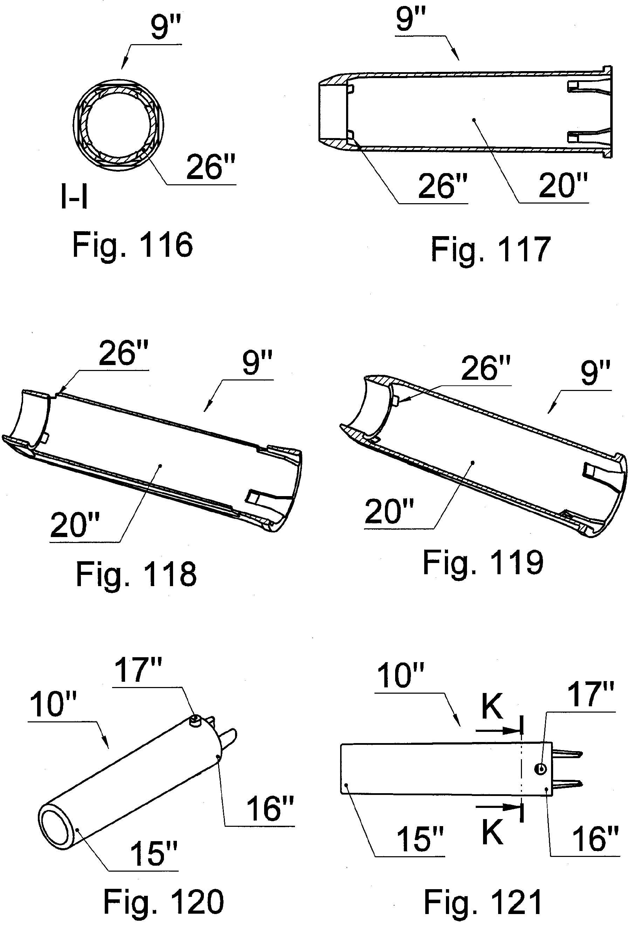

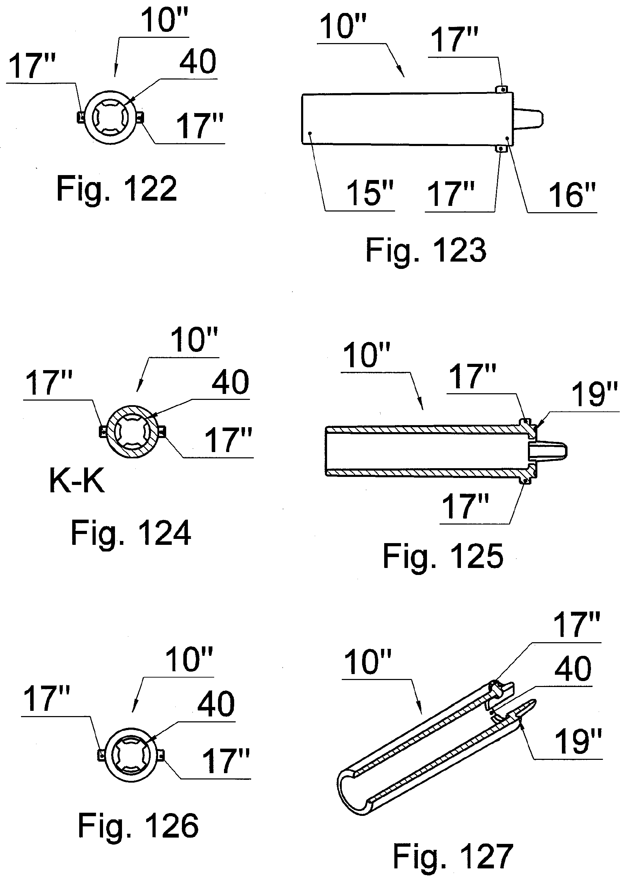

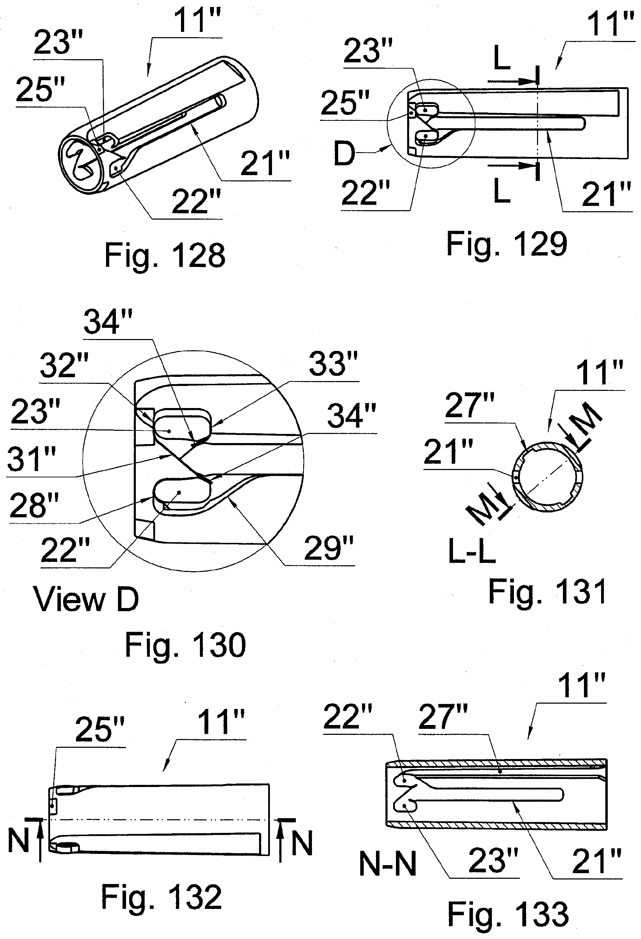

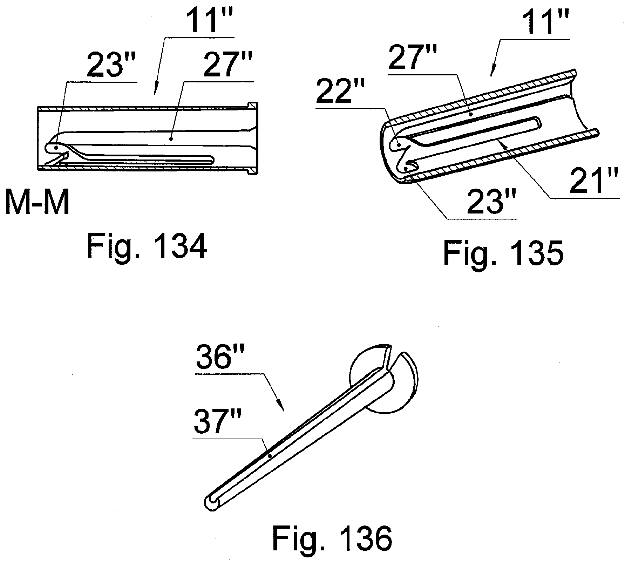

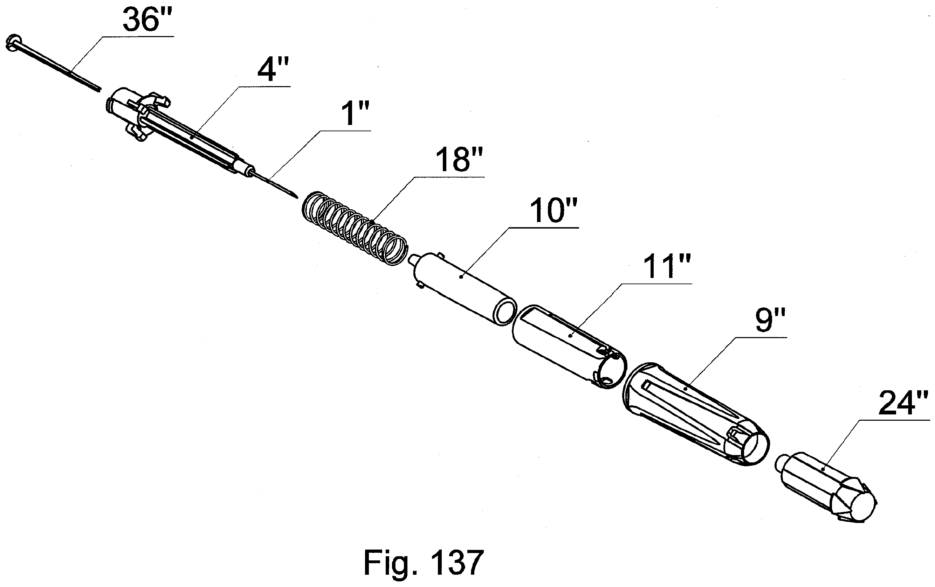

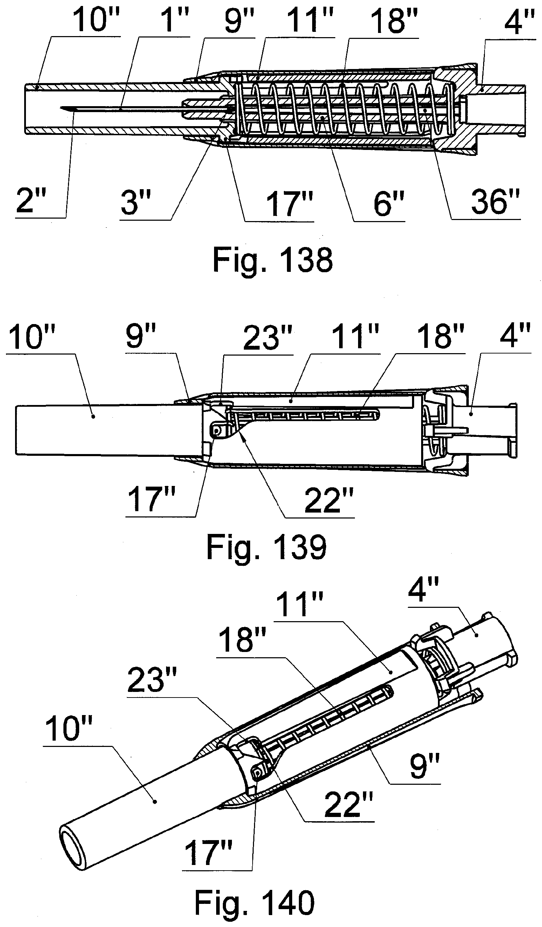

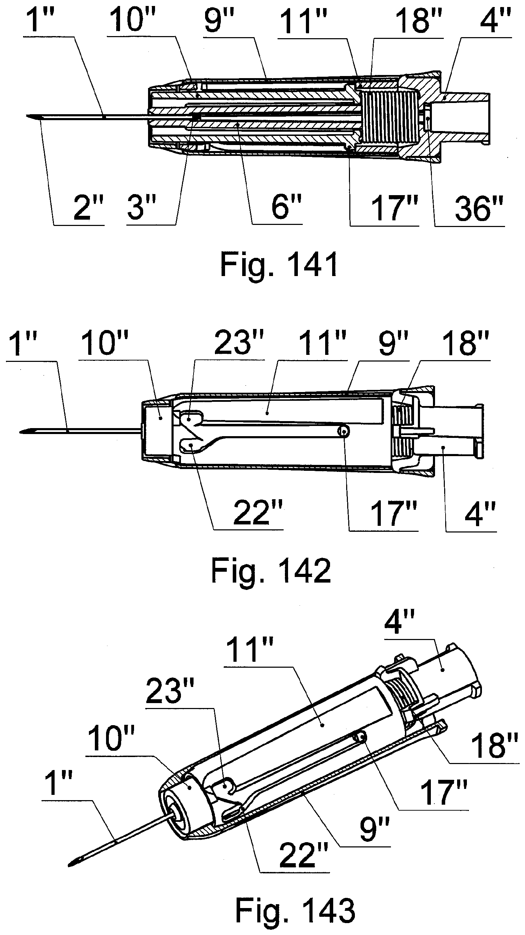

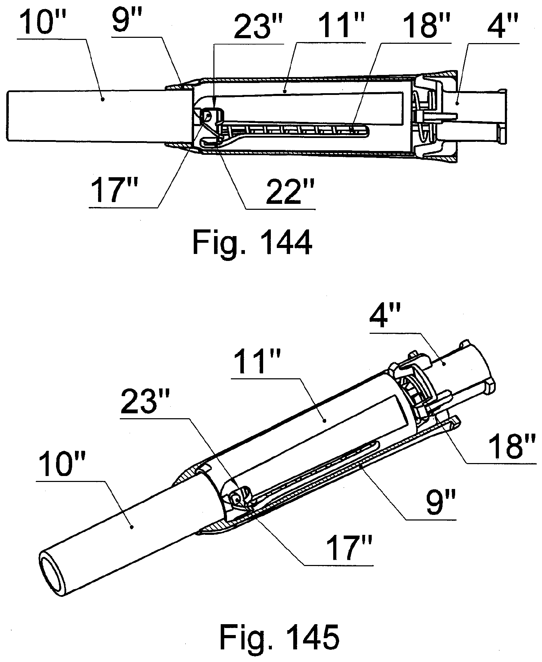

Fig.96 shows the complete device of third exemplary embodiment in a ready-to-use state (without a cover) in a side view, and Fig.97, Fig.98 and Fig.99 show the same device in a rear view, in front view (from the needle side) and in axonometric front view, respectively. Fig.100 shows the device of Fig.96 with the housing removed and Fig.101 shows the same device after additional removal of the slider and the rotary sleeve. Fig.102 shows the device of Fig.96 with the cover applied and Fig.103 shows the same device in a longitudinal section. Fig.104, Fig.105, Fig.106 and Fig.107 show the hub of the device of Fig.96, in a side view, in a front view, in an axonometric front view and in the same view with cut-away, respectively. Fig.108 shows an axonometric view of the hub of Fig.106 with a needle placed therein, Fig.109 shows a side view of the hub with the needle, Fig.110 shows a longitudinal section of the hub with the needle, while Fig.111 shows the same hub with the needle in a cut-away axonometric view. Fig.112 shows an axonometric front view of the housing of the device of Fig.96. Figures from Fig.113 to Fig.119 show the housing of Fig. 112 in a side view, in a front view, in a first longitudinal section, in a cross-section, in a second longitudinal section, in an axonometric view of the first longitudinal section and in an axonometric view of the second longitudinal section, respectively. Fig.120 shows a front axonometric view of the slider of the device of Fig.96. Figures from Fig.121 to Fig.127 show the slider of Fig.120 in a first side view, in a front view, in a second side view, in a cross-section, in a longitudinal section, in a rear view and in a cut-away axonometric view, respectively. Fig.128 shows an axonometric view from the front of the rotary sleeve of the device of Fig.96. Figures from Fig.129 to Fig.135 show the sleeve of Fig.128 in a first side view, in an enlarged part of the first side view, in a cross-section view, in a second side view, in a first longitudinal section, in a second longitudinal section and in an axonometric view of the first longitudinal section, respectively. Fig.136 shows an axonometric view of the reduction insert of the hub of Fig.104. Fig.137 shows the device of Fig.102 in an exploded view. Fig.138, Fig.139 and Fig.140 show the device of Fig.96 in first phase, i.e. ready for insertion. Fig.141, Fig.142 and Fig.143 show the device of Fig.96 after insertion into the patient's body (not shown), i.e. at the end of second phase and in third phase, while Fig.144 and Fig.145 show the same device in a b locked state, i.e. in fourth phase.

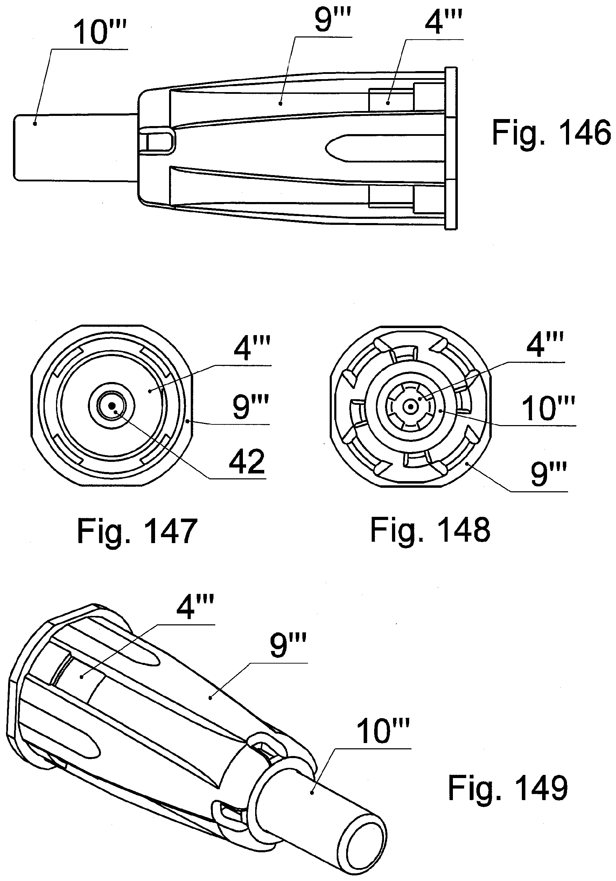

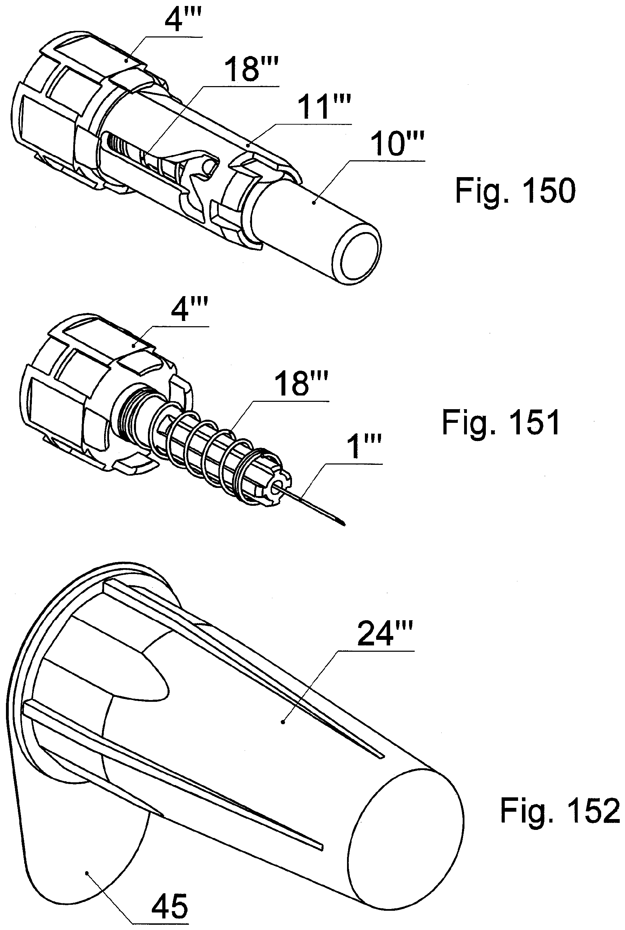

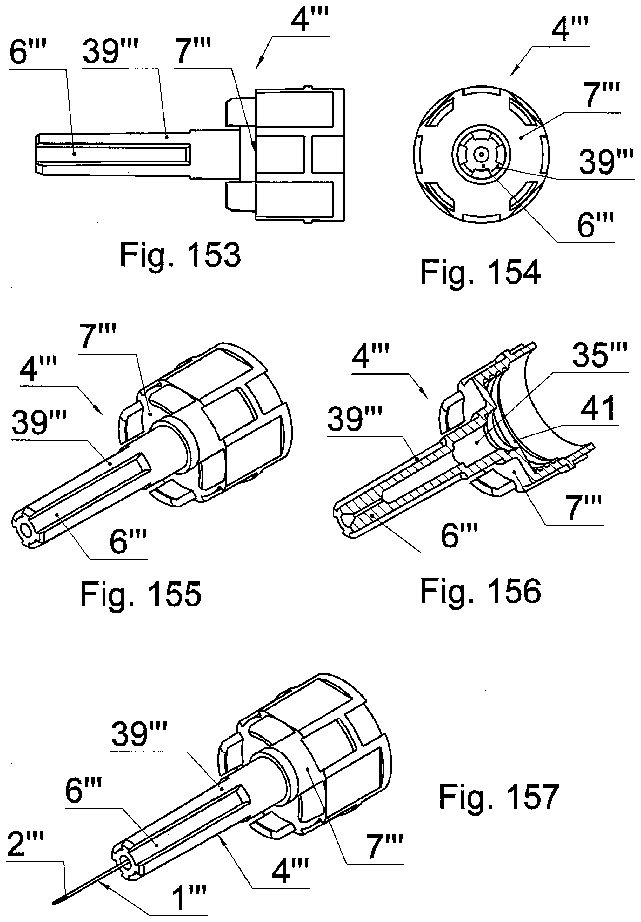

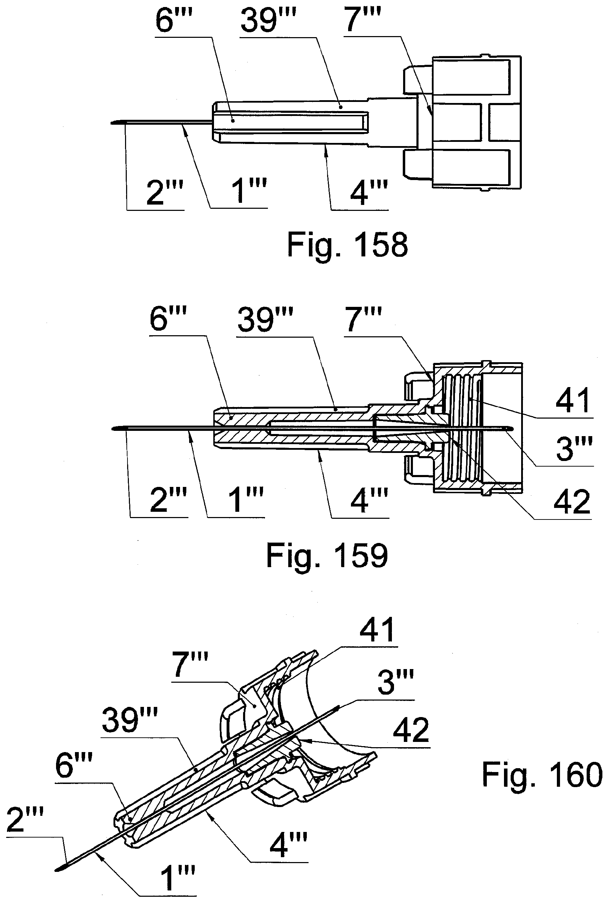

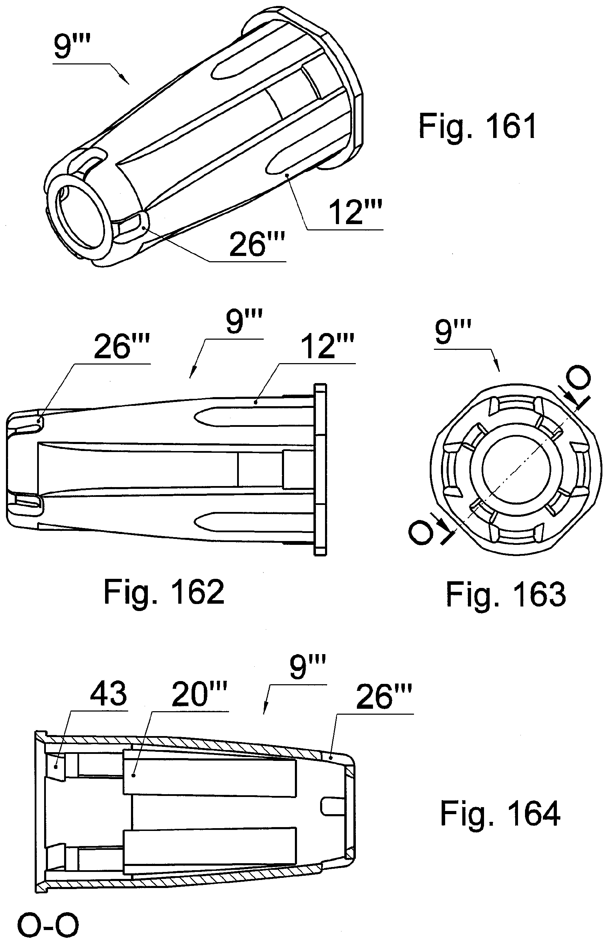

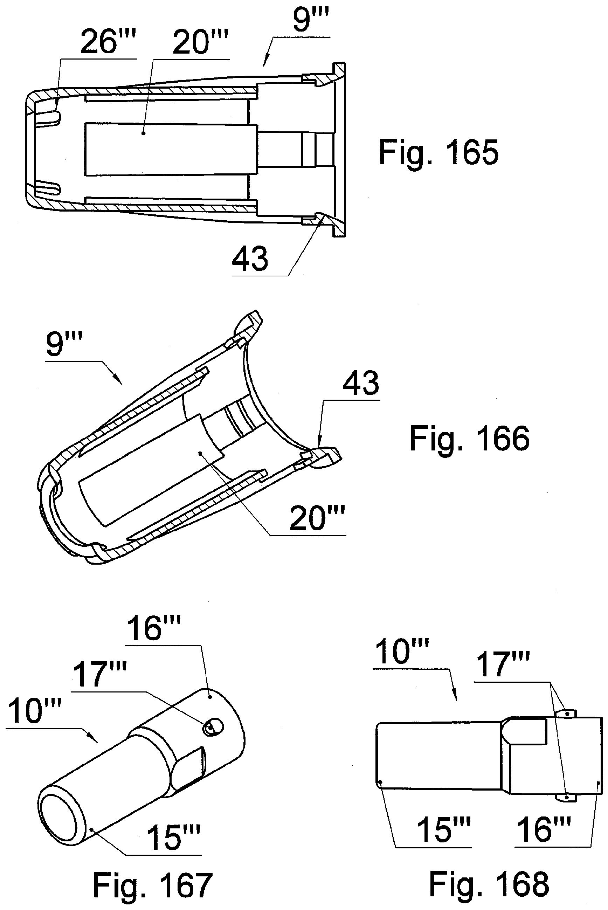

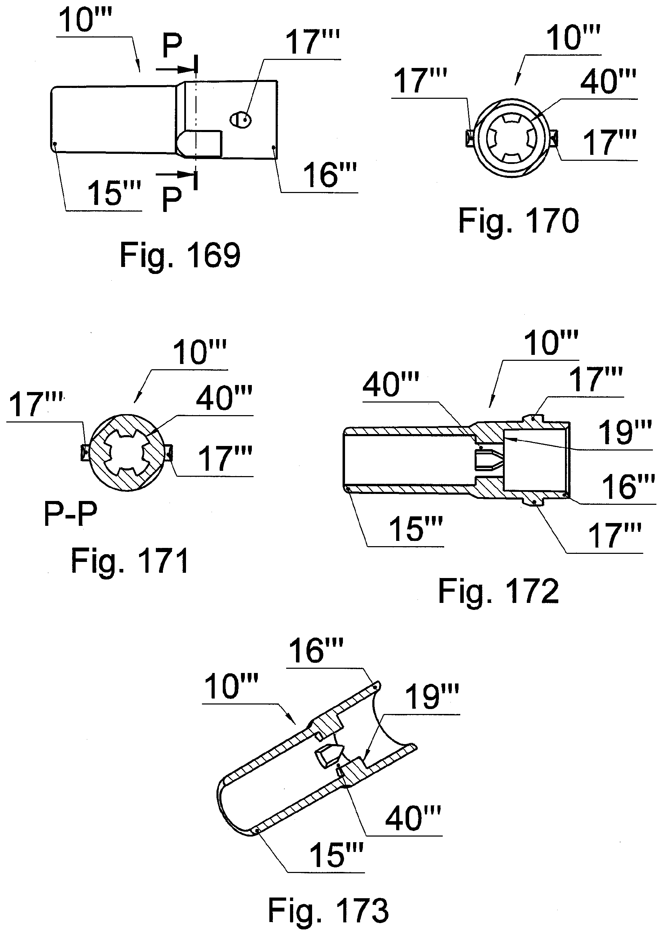

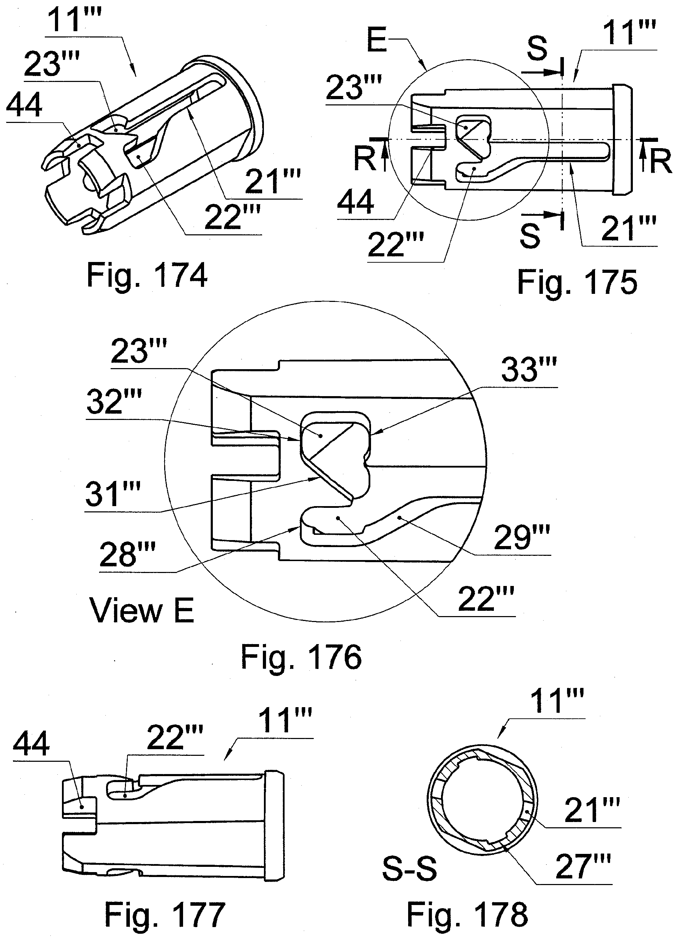

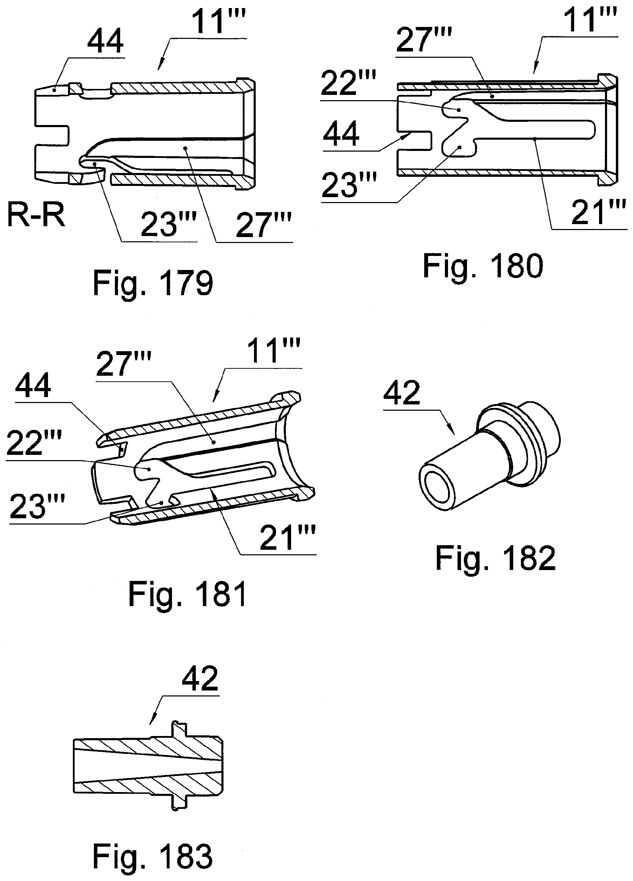

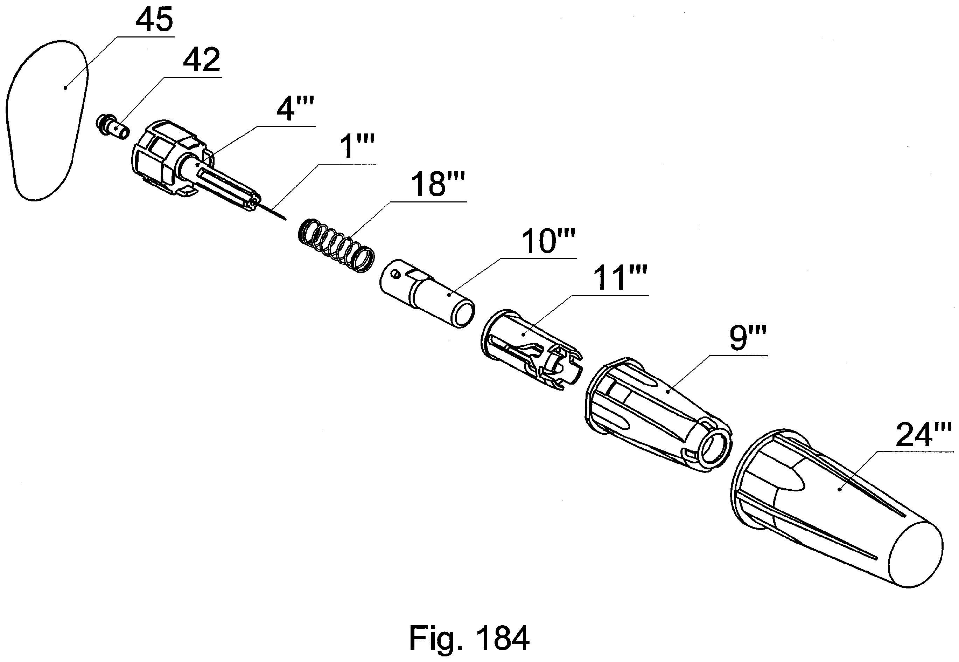

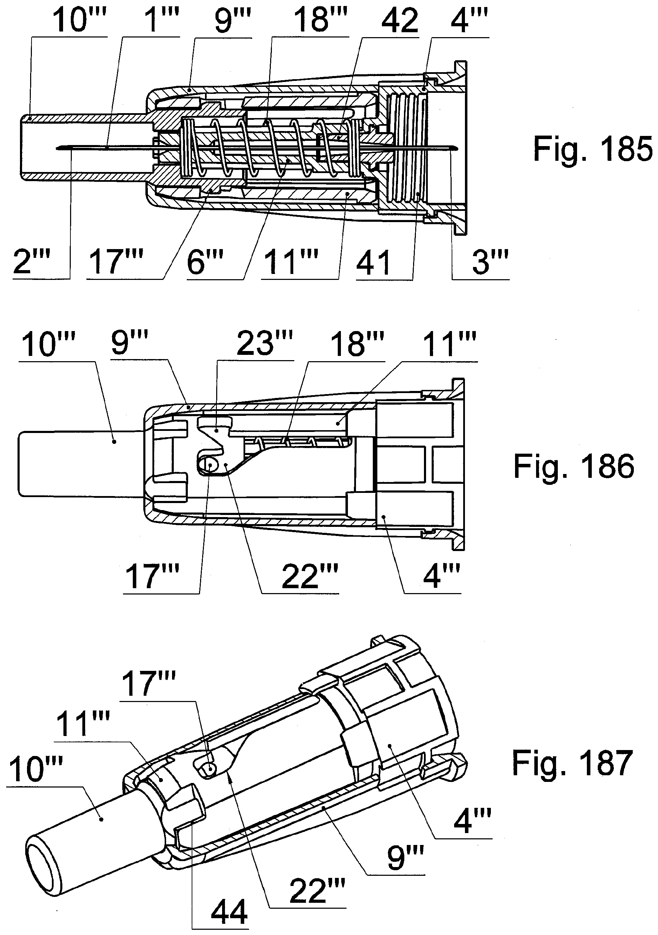

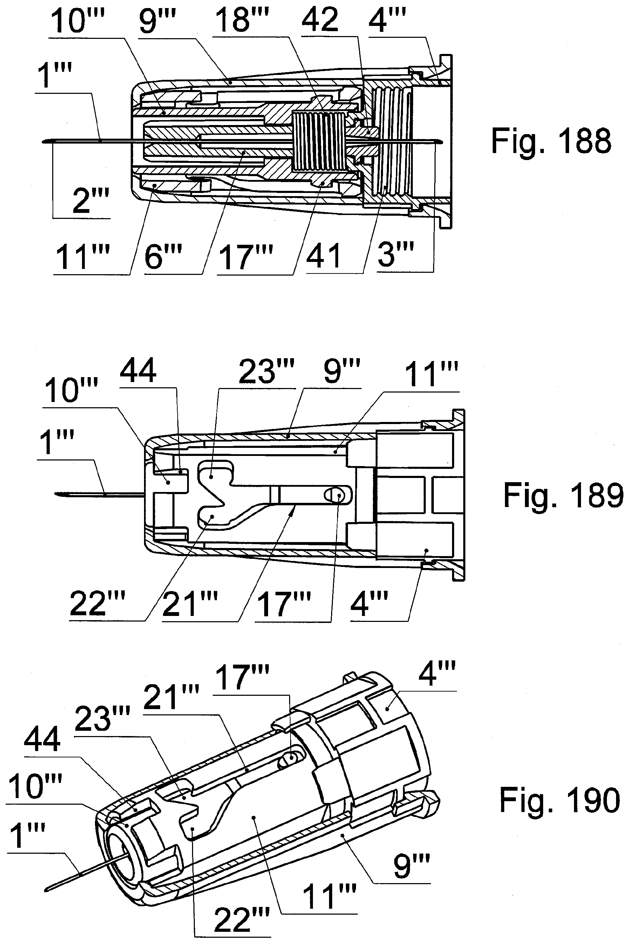

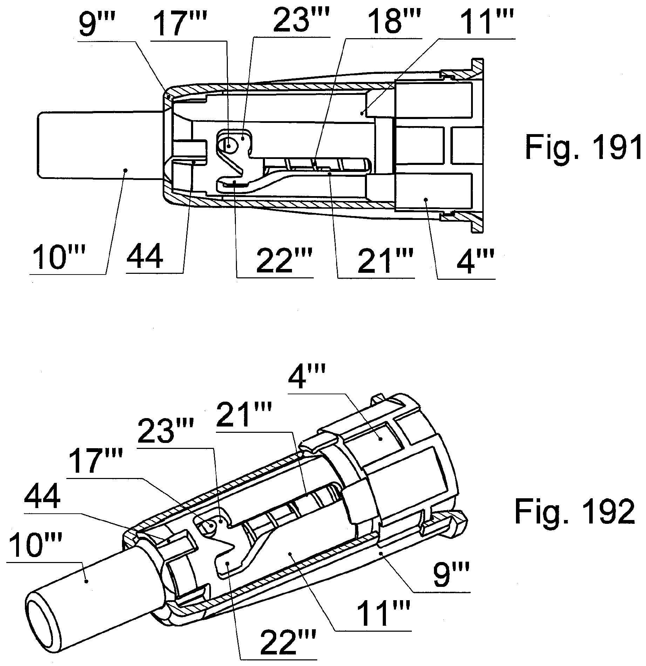

Fig.146 shows the complete device of the fourth exemplary embodiment in a ready-to-use state (without the cover) in a side view, and Fig.147, Fig.148 and Fig.149 show the same device in a rear view; in front view (from the needle side) and in an axonometric front view, respectively. Fig.150 shows the device of Fig.146 with the housing removed and. Fig.151 shows the same device after additional removal of the slider and the rotary sleeve. Fig.152 shows the device of Fig.146 with the cover applied and closed with a seal. Fig.153, Fig.154, Fig.155 and Fig.156 show the hub of the device of Fig.146, in a side view, in a front view, in an axonometric front view and in an axonometric view of the longitudinal section, respectively. Fig.157 shows an axonometric view of the hub of Fig.153 with a needle placed therein, Fig.158 shows a side view of the hub with the needle, Fig.159 shows a longitudinal section of this hub with the needle and needle stabiliser, while Fig.160 shows an axonometric view of the section of Fig.159. Fig.161 shows an axonometric front view of the housing of the device of Fig.146. Figures from Fig.162 to Fig.166 show the housing of Fig. 161 in a side view, in a front view, in a first longitudinal section, in a second longitudinal section and in an axonometric view of the second longitudinal section, respectively. Fig.167 shows an axonometric front view of the slider of the device of Fig.146. Figures from Fig.168 to Fig.173 show the slider of Fig.167 in a first side view, in a second side view, in a front view, in a cross-section, in a longitudinal section and in an axonometric view of this longitudinal section, respectively. Fig.174 shows an axonometric front view of the rotary sleeve of the device of Fig.146. Figures from Fig.175 to Fig.181 show the sleeve of Fig.174 in a first side view, in an enlarged part of the first side view, in a second side view, in a cross-section, in a first longitudinal section, in a second longitudinal section and in an axonometric view of the second longitudinal section, respectively. Fig.182 shows an axonometric view of the second needle end stabiliser and Fig.183 shows a longitudinal section of the stabiliser. Fig.184 shows the device of Fig.152 in an exploded view. Fig.185, Fig.186 and Fig.187 show the device of Fig.146 in first phase, i.e. ready for insertion. Fig.188, Fig.189 and Fig.190 show the device of Fig.146 after insertion into the patient's body (not shown), i.e. at the end of second phase and in third phase, while Fig.191 and Fig.192 show the same device in a blocked state, i.e. in fourth phase.

Four exemplary embodiments of the invention are described in detail below

### Example I

The first exemplary embodiment of the invention is shown in the figures from Fig.1 to Fig.49. The device comprises an elongated needle 1 in the form of a metal tube with a diameter of 0.5 mm; however using a needle 1 with a diameter from 0.3 to 1.3 mm will not affect the operation of the device. The first end 2 of the needle 1, intended for insertion into the patient's body, is sharpened obliquely in the known manner. The second end 3 of the needle 1 is placed in the hub 4 made with plastic injection moulding technology. Placement of needle 1 in the hub 4 can occur already at the stage of hub 4 forming, or by gluing or pressing into an opening in the finished hub 4. The hub 4 allows a channel in the needle 1 to be connected with a vessel for a substance to be injected or a vessel for receiving samples from the patient, for example with a known syringe. In this example the hub 4 is equipped with a socket 5 of the known system for connecting medical instruments, e.g. needles, syringes and catheters, with the trade name "luer lock". The needle 1 is mounted in an axial central member 6, which forms an integral part of the hub 4. The hub 4 has a base 7, perpendicular to the central member 6 and located on the side of the element 6 opposite the free end of the needle 1, and a circumferential wall 8 extending from the outer edge of the base 7 towards the first end 2 of the needle 1. A plastic housing 9 is inserted over the circumferential wall 8 and snap-mounted to the hub 4. The housing 9 holds a slider 10 and a rotary sleeve 11. The housing 9 has four longitudinal projections 12 on the outside, which make it easier for the user to detach the hub 4 from the above-mentioned syringe or another medical instrument with a similar function. The slider 10 has substantially a tubular shape and can move along the axis of the needle 1. The movement of slider 10 begins from the stable initial position before use of the device and ends in the stable end position after insertion. In both of these stable positions of slider 10, the first end 2 of the needle 1 is secured, i.e. shielded by the slider 10, which protects the user from accidental injuries. The slider 10 has two linear guides 13 on its outer surface, working in combination with the guide notches 14 of the housing 9 located at the end of the housing 9 opposite to the hub 4. The guides 13 and the notches 14 work together, preventing rotation of the slider 10 with respect to the housing 9. The first end 15 of the slider 10 in the initial and end positions protrudes from the housing 9, while the second end 16 of the slider 10 is always inside the housing 9. In the vicinity of the second end 16, the slider 10 features two pins 17 located transversely in relation to the axis of the needle 1 and constituting elements of a mechanism blocking the slider's 10 movements in the position most extended out of the housing 9 after the first end 2 of the needle 1 is removed from the patient's body. In the described example, the pins 17 constitute an integral part of the slider 10 made in one injection moulding process from a transparent plastic. The transparency of the slider 10 material allows continuous observation of the first end 2 of the needle 1, therefore increasing insertion precision. This stems from the fact that the user may, by looking through the transparent material of the slider 10, control the presence or absence of the needle 1, its appearance and co-axial position in relation to the socket 5, as well as check the flow and remove air from the needle 1 prior to insertion. Inside the slider 10 there is a longitudinal resilient means in the form of a metal helical spring 18. The first end of the spring 18 is supported against the inner retaining surface 19 of the slider 10 and the second end of the spring 18 surrounds the central member 6 and is supported on the retaining surface of the hub 4, i.e. on a fragment of the base 7 located between the central member 6 and the circumferential wall 8. Placing the spring 18 in the slider 10 reduces the overall length of the device. In the housing 9 there is a cylindrical channel 20 with an axis parallel to the axis of the needle 1. The previously mentioned rotary sleeve 11, which has a tubular shape, is movably mounted in the channel 20. The longitudinal axis of the sleeve 11 is in line with the axis of the needle 1. In the side wall of the sleeve 11 there are two linear notches 21 running along the axis of the sleeve 11, in which the pins 17 of the slider 10 are guided. Each of the notches 21 of the sleeve 11 has, at the end, a branching similar in shape to the letter "V", located adjacent to the end closer to the first end 2 of the needle 1. Each of these branches consists of two bays, a start bay 22 and a blocking bay 23. The notches 21 and the bays 22 and 23 constitute further elements of the mechanism blocking the slider's 10 axial movement. The device according to the invention is distributed with a cover 24 installed, which allows the device to be safely operated during connecting to a chosen medical instrument. The device according to the invention is also equipped with a use indicator. It consists of four indicator fields 25 with a high-visibility colour, located on the external surface of the sleeve 11 and of four windows 26 in the housing 9. In the state of the device before its use, the indicator fields 25 are invisible, while after the rotation of the sleeve 11 as a result of the needle insertion described below, they begin to be visible through the windows 26. In one of variants, transparent panes can be placed in the windows 26, preventing the user from interfering with the state of the device's use indicator. Assembly of the device according to the invention starts with the mounting of the needle 1 in the hub 4. The needle 1 may be covered with a known lubricating agent that reduces friction during insertion the needle 1 into the patient's body and reduces the pain perceptible during this process. A spring 18 is placed in axis of the hub 4 with the needle 1, resting against the base 7 of the hub 4. The slider 10 is placed in the sleeve 11 by introduction into the sleeve 11 of the first end 15 of the slider 10 from the side opposite to the end near which the bays 22 and 23 are located. The pins 17 are inserted into two inner assembly recesses 27 of the sleeve 11 and this insertion is stopped before the pins 17 reach the start bays 22. The pins 17 are guided in the assembly recesses 27 with an interference fit. Thanks to this interference fit, in the sub-assembly thus created, consisting of the slider 10 and the sleeve 11, the mutual position of the two elements is stable. This sub-assembly is then introduced with the first end 15 of the slider 10 into the wider end of the housing 9, i.e. into the end of the housing 9 that contains means for connecting it to the hub 4, until the guides 13 of the slider 10 are inserted into the guide notches 14 of the housing 9. The three-component sub-assembly formed in this manner, consisting of the housing 9, the slider 10 and the sleeve 11, is mounted to the previously assembled sub-assembly consisting of the needle 1, the hub 4 and the spring 18 until the housing 9 clicks on the circumferential wall 8 of the hub 4, with care that the free end of the spring 18 rests on the retaining surface 19 of the slider 10. Partial insertion of the slider 10 into the sleeve 11 makes it possible to accurately assess the second end 2 of the needle 1. If this assessment is successful, the slider 10 is pulled out of the housing 9 until the pins 17 enter the start bays 22. Finally, the cover 24 is applied to the slider 10 and the housing 9.

The operation of the above-described exemplary device according to the invention, is described below:

### Phase 1 - preparing the safety needle device for use (Fig.43 and Fig.44)

In this phase, the user removes the device from the sterile blister not shown in the drawing and places the device on the selected medical instrument by pressing the fitting cone of the said instrument into the socket 5, and then mutually rotating to couple socket 5 with the corresponding fitting element of the medical instrument. The user removes the cover 24 from the device and then checks the state of the use indicator through the window 26 in the housing 9, i.e. the visibility of the indicator fields 25 on the sleeve 11. In this phase, indicator fields 25 should not be visible in the windows 26 of the housing 9. The user also checks, by looking through the transparent slider 10, the visual state of the first end 2 of the needle 1 and its patency. Under the pressure of the spring 18, the slider 10 is completely extended and completely covers the needle 1, while the pins 17 are in the start bays 22 and under the pressure of the spring 18 are supported against the start retaining surfaces 28.

### Phase 2 - insertion

The user brings the first end 15 of the slider 10 to the intended insertion point in the patient's body and presses the housing 9 against the body, inserting the needle 1 to the required depth. If necessary, the user presses the device housing 9 against the body as far as possible, using the maximum insertion depth offered by the device. The slider 10 starts longitudinal movement inside the housing 9 and gradually compresses the spring 18. As a result of the movement of the slider 10 being initiated, the pins 17 lose contact with the start retaining surfaces 28. Then they exit the start bays 22 and exert pressure on the first guiding surfaces 29 of the sleeve 11. Under this pressure, the sleeve 11 rotates around the axis of the needle 1 and the pins 17 move into the linear notches 21. Rotation of the sleeve 11 is a result of the oblique slope of the first guiding surfaces 29 and initiates the operation of the mechanism blocking movement of the slider 10, and prevents its return to the initial state. This occurs even when the slider 10 is slightly retracted into the housing 9, which may even not lead to actual insertion of the needle 1 into the patient's body. The length to which the pins 17 are inserted into the linear notches 21 corresponds to the depth of needle 1 insertion. Release of the pins 17 from the start bays 22 tightens the spring 18, which causes their presence in the notches 21 to be unstable, as a loss of pressure on the slider 10 will always cause the mechanism to block reuse the device according to the invention. In the described example, the hollow part of the slider 10 has a narrowed section 30 supporting the needle 1 and increasing the precision of its insertion.

### Phase 3 - injection/collection (Fig.45, Fig.46 and Fig.47)

Upon reaching the required insertion depth, the user holds the device in one position against the body and injects a set dose of the cosmetic and/or pharmaceutical composition or collects a sample of the body tissue/fluid, by pushing on the plunger of the external injection device completely, according to the manufacturer's instructions or, respectively, by pulling the plunger of the collection device.

### Phase 4 - withdrawal of the needle from the patient's body and device blocking

In the next phase, the user moves the safety needle device away from the patient's body, which results in the removal of the first end 2 of the needle 1 from the body. When the needle 1 is removed from the body, the slider 10 automatically slides out of the housing 9, which occurs under the pressure of the spring 18, i.e. the part of the needle 1 removed from the body is automatically covered by the slider 10 all the time. As the slider 10 slides out, the pins 17 move in the recess 21 until they reach the second guiding surfaces 31 of the sleeve 11. The oblique placement of this surfaces causes further rotation of the sleeve 11 and insertion of the pins 17 into the blocking bays 23 and their placement against the first blocking retaining surfaces 32. In this state, subsequent depression of the slider 11 will cause the pins 17 to block against the second blocking retaining surfaces 33 (Fig.48 and Fig.49), preventing its further movement into the housing 9. The possible movement range of the pins 17, and therefore of the slider 10, between the first and second blocking retaining surfaces 32 and 33 is too small for the first end 15 of the slider 10 to expose the first end 2 of the needle 1. Since the guides 13 and the notches 14 stop rotation of the slider 10 in relation to the housing 9, the user cannot move the slider 10 to its initial state without destructive interference in the device. The sleeve 11 may have elastic safety tabs 34 at the exit from the start bay 22 and at the entry to the blocking bay 23. Rotation of the sleeve 11 caused by the pressure of the pins 17 exerted, in turn, on the first and second guiding surface 29 and 31, causes the indicator fields 25 to appear in the windows 26, informing the user that the device is no longer usable. A blocked device can be safely detached from the medical instrument, with the longitudinal projections 12 facilitating device rotation, making it easier to disconnect it from the instrument.

### Example II

The second exemplary embodiment of the invention, shown in the figures from Fig.50 to Fig.95, has a design similar to the one of the first example. Corresponding elements of this device have the same numbers in the drawing and in the description as in the first example, but supplemented with an apostrophe ('). The hub 4' has a much longer central member 6' and four hooks 38 for attaching the housing 9', while the cover 24' is much shorter. In this example the second end 3' of the needle 1' is mounted at the very end of the central member 6', equipped with a long channel 35. In the case of injections of expensive drugs, the volume of the channel 35 can be reduced by means of a reduction insert 36 with a longitudinal channel 37. In this example, the housing 9' is mounted on the hub 4' via its four hooks 38. The narrow section 30' of the slider 11' is at its first end 15'. In this example, in the device's initial state, the first end 2' of the needle 1' protrudes from the slider 10', making it much easier to start the insertion of the needle 1' in a precisely selected place on the patient's body. The sleeve 11' has two straight notches 21', but the position of the start bay 22' is different than in the first example. The blocking bay 23' is at the same end of the linear notch 21' as in the first example. Meanwhile, the start bay 22' is located on the side of the notch 21' and closer to the hub 4' than the blocking bay 23'. The length of the slider 10' is selected so that when the pins 17' are in the start bay 22', the length of the visible end of the needle 1' is a few millimetres, whereas when the pins 17' reach the blocking bay 23', the first end 15' of the slider 10' completely covers the first end 2' of the needle 1', even accounting for the play of the pins 17' between the first and second blocking retaining surface 32' and 33'. In this example the mechanism for indicating use of the device differs. The indicator field 25' is a high visibility strip on the slider 10', which becomes visible to the user only when the pins 17' are in the blocking bays 23'. The first phase of operation described in the previous example is shown in Fig.87, Fig.88 and Fig.89. The device in the third phase is shown in Fig.90, Fig.91 and Fig.92, while Fig.93, Fig.94 and Fig.95 show the device in the fourth phase of operation.

### Example III

The third exemplary embodiment of the invention, shown in the figures from Fig.96 to Fig.145, combines the technical features of the two preceding examples. Corresponding elements of this device have the same numbers as in the first example, but supplemented with a double apostrophe ("). The hub 4" differs from the hub 4' only in the presence of four longitudinal guides 39 feature d on the elongated central element 6" and inserted into the internal guide notches 40 of the slider 10". The guides 39 and the notches 40 have the same role as the guides 13 and notches 14 in the first example, i.e. they block rotation of the slider 10" in relation to the housing 9". Also in this example, the volume of the central member's 6" channel 35" can be reduced using the 36" reduction insert with channel 37". The sleeve 11" with linear notches 21", start bays 22" and blocking bays 23" is very similar to the sleeve 11 described in the first example. In this example the part of the slider 10" visible on the outside of the housing 9" is a smooth transparent tube, which makes it possible to accurately observe the needle 1" from each side. The first phase of operation described in the first example is shown in Fig.138, Fig.139 and Fig.140. The device in the third operational phase is shown in Fig.141, Fig.142 and Fig.143, while Fig.144 and Fig.145 show the device in the fourth phase of operation.

### Example IV

The fourth exemplary embodiment of the invention is shown in the figures from Fig.146 to Fig.192. Its design is the closest to the solution of the third example. Corresponding elements of this device have the same numbers as in the previous examples, but supplemented with a triple apostrophe (‴). The hub 4‴ differs from the previous in that it has a thread 41 at its second end, which allows attachment to a dedicated insulin injection device, widely known as an "insulin pen". The needle 1‴ is sharpened in the known manner on its first 2‴ and second 3‴ end. The first end 2‴ of the needle 1"' is used for insertion into the patient's body, as in the previous examples. The second end 3‴ of the needle 1‴ protrudes from the hub 4‴ on the side opposite to the first end 2‴ and is intended for insertion into the insulin ampoule (not shown), when the device is being mounted on the aforementioned ,pen". In the slightly different shaped channel 35‴ of the hub 4‴ is placed a stabiliser 42, supporting the second end 3‴ of the needle 1‴ and assuring stability during insertion into the ampoule. The housing 9‴ is mounted on the hub 4"' via its four internal latches 43. The operational principle of the device use indicator is very similar to the corresponding indicator in the first example. The housing 9‴ has four windows 26"' through which the outer surface of the rotary sleeve 11‴ can be observed. Instead of the separate markers 25, the entire outer surface of the sleeve 11‴ has a colour that contrasts with the colour of the housing 9‴, so, for example, it is made of a material with such a contrasting colour. In addition, at the end of the sleeve 11‴ there are four indicating indents 44, visible in windows 26‴ when the device is ready to use, i.e. in the first phase. The movement of the pins 17‴ of the slider 10‴ from the start bays 22‴ of the sleeve 11‴ to its blocking bays 23‴ causes rotation of the sleeve 11‴, as described in detail in the first example, and therefore the appearance of its contrast-coloured walls in the windows 26‴. The device in this example does not require a separate package to ensure sterility, because after the device is placed in the cover cover 24‴ it can be sealed with a seal 45 attached in a known manner to the rim of the cover 24‴ (Fig.152). The protruding seal lip 45 makes it possible to tear away the seal and remove the device from the cover 24"'.

Each of the examples described above can be modified according to one's needs. For example, the housing 9 can be installed on the hub 4 using an adhesive. Instead of a metal spring 18, a plastic spring can be used, thus simplifying the disposal process, as raw material segregation is not required. The "luer lock" socket 5 can be replaced by any other connector used in medical equipment, e.g. a "luer slip". Furthermore, the placement of the start bay 22 and the blocking bay 23 on the sleeve 11 is independent of the method of blocking the slider's 10 rotation in relation to the housing 9.

**List of designations**

| | | | |
|---|---|---|---|
| 1. | needle | 32 | first blocking retaining surface |
| 2. | first end of the needle | 33 | second blocking retaining surface |
| 3. | second end of the needle | 34 | safety tab |
| 4. | hub | 35 | central member channel |
| 5. | socket | 36 | reduction insert |
| 6. | central member | 37 | channel of the reduction insert |
| 7. | base | 38 | hub hook |
| 8. | circumferential wall | 39 | guide |
| 9. | housing | 40 | guide notch |
| 10 | slider | 41 | hub thread |
| 11 | rotary sleeve | 42 | needle stabiliser |
| 12 | longitudinal projection | 43 | housing latch |
| 13 | guide | 44 | indicator indent |
| 14 | guide notch | 45 | seal |
| 15 | first end of the slider | | |
| 16 | second end of the slider | | |
| 17 | pin | | |
| 18 | helical spring | | |
| 19 | retaining surface | | |
| 20 | cylindrical channel | | |
| 21 | linear notch | | |
| 22 | start bay | | |
| 23 | blocking bay | | |
| 24 | cover | | |
| 25 | indicator field | | |
| 26 | window | | |
| 27 | assembly recess | | |
| 28 | start retaining surface | | |
| 29 | first guiding surface | | |
| 30 | internal narrow section of the slider | | |
| 31 | second guiding surface | | |

## Claims

1. Safety needle device for medical instruments, comprising:
- an elongated needle (1, 1', 1", 1‴) with an in-axis channel, with the first end (2, 2', 2'', 2‴) to be inserted into the patient's body and embedded in a hub (4, 4', 4'', 4‴) in the area of its (1, 1', 1" , 1‴) second end (3, 3', 3'', 3‴), with the hub (4, 4', 4", 4‴) provided with means for connecting the needle's (1, 1', 1", 1‴) channel with a vessel for supplying an injectable substance or a vessel for receiving a sample of the patient's tissue;
- a housing (9, 9', 9'', 9‴) fixed immovably on the hub (4, 4', 4'', 4‴) and surrounding the needle (1, 1', 1'', 1‴) along a part of its length;
- a hollow slider (10, 10', 10", 10‴) surrounding the needle (1, 1', 1", 1‴) and moving longitudinally and freely along the needle's (1, 1', 1", 1‴) axis, with its first end (15, 15', 15", 15‴) directed towards the outside of the housing (9, 9', 9", 9"') and the second end (16, 16', 16", 16‴) moving inside the housing (9, 9', 9", 9‴) and directed towards the hub (4, 4', 4", 4‴), as well as means preventing the slider from rotating (13, 13', 14, 14', 39, 39‴, 40, 40‴) relative to the housing (9, 9', 9", 9‴) and the second end (16, 16', 16", 16‴) of the slider (10, 10', 10", 10"') from sliding out of the housing (9, 9', 9", 9‴); the slider (10, 10', 10", 10‴) being movable from an initial position before injection to an end position after injection
- a longitudinal resilient means (18, 18', 18", 18‴ ) located inside the housing (9, 9', 9", 9‴) and configured to be gradually compressed during the longitudinal movement of the slider (10, 10', 10", 10‴) inside the housing (9, 9', 9", 9‴), with its first end backed against the slider (10, 10', 10", 10‴) and with its second end backed against the hub (4, 4', 4", 4‴), as well as being at the lowest level of tension when the slider (10, 10', 10", 10‴) is most extended with its first end (15, 15', 15", 15‴) from the housing (9, 9', 9", 9‴);
- means for blocking the slider's (10, 10', 10'', 10‴) movement in the position most extended out of the housing (9, 9', 9", 9‴) after the first end (2, 2', 2'', 2‴) of the needle (1, 1', 1", 1‴) is removed from the patient's body;
- in the housing (9, 9', 9'', 9‴) there is a cylindrical channel (20, 20', 20'', 20‴) with its axis parallel to the axis of the needle (1, 1', 1'', 1‴);
- in the cylindrical channel (20, 20', 20'', 20‴) of this housing (9, 9', 9", 9‴) is located a rotating sleeve (11, 11', 11", 11‴) surrounding the needle (1, 1', 1'', 1‴) and configured to rotate around the axis of the needle upon longitudinal movement of the slider (10, 10', 10'', 10‴), a longitudinal resilient means (18, 18', 18'', 18‴) and at least the second end (16, 16', 16'', 16‴) of the slider (10, 10', 10'', 10‴);
- in the side wall of the rotating sleeve (11, 11', 11'', 11‴) there is at least one linear notch (21, 21', 21", 21"') with its axis along the needle's (1, 1', 1", 1‴) axis, having a first end and a second end as well as a start bay (22, 22', 22", 22‴) and a blocking bay (23, 23', 23", 23‴), wherein the first end of the linear notch (21, 21', 21", 21‴) is closer to the first end (2, 2', 2", 2‴) of the needle (1, 1', 1", 1‴) than the second end of the linear notch (21, 21', 21", 21‴);
- in the vicinity of the second end (16, 16', 16", 16‴) of the slider (10, 10', 10", 10"') there is at least one pin (17, 17', 17", 17‴) located transversely to the needle's (1, 1', 1", 1‴) axis and coupled mechanically to the linear notch (21, 21', 21", 21"') in the side wall of the rotating sleeve (11, 11', 11", 11‴),
- wherein under the pressure of the longitudinal resilient means (18, 18', 18", 18‴), the slider (10, 10', 10", 10‴) is completely extended and completely covers the needle (1, 1', 1", 1"'), while the pin (17, 17', 17", 17‴) is in the start bay (22, 22', 22", 22‴) and under the pressure of the longitudinal resilient means (18, 18', 18", 18‴) is supported against a start retaining surface (28, 28', 28", 28‴);
- wherein, when the needle (1, 1', 1", 1‴) is inserted and as a result of the movement of the slider (10, 10', 10'', 10‴) being initiated, the pin (17, 17', 17'', 17‴) loses contact with the start retaining surface (28, 28', 28'', 28‴) and exits the start bay (22, 22', 22", 22‴) and exerts pressure on a first guiding surface (29, 29', 29'', 29‴) of the rotating sleeve (11, 11', 11", 11‴) due to an oblique slope of the first guiding surface (29, 29', 29", 29‴), causing the rotating sleeve (11, 11', 11", 11‴) to rotate around the axis of the needle (1, 1', 1", 1‴) and the pin (17, 17', 17'', 17‴) moves into the linear notch (21, 21', 21", 21‴);
- wherein, when the needle (1, 1', 1", 1‴) is withdrawn and as the slider (10, 10', 10'', 10‴) slides out, the pin (17, 17', 17'', 17‴) moves in the linear notch (21, 21', 21", 21‴) until it reaches a second guiding surface (31, 31', 31'', 31‴) of the rotating sleeve (11, 11', 11'', 11‴), said surface having an oblique placement causing further rotation of the rotating sleeve (11, 11', 11'', 11‴) and insertion of the pin (17, 17', 17'', 17‴) into a blocking bay (23, 23', 23'', 23‴) and its placement against a first blocking retaining surface (32, 32', 32'', 32‴) so subsequent depression of the slider (10, 10', 10'', 10‴) will cause the pin (17, 17', 17", 17‴) to block against a second blocking retaining surface (33, 33', 33", 33‴), preventing its further movement into,
- the rotation of the slider (10, 10') with respect to the housing (9, 9') is prevented by the slider (10, 10') having two liner guides (13, 13') on its outer surface, working in combination with guide notches (14, 14') of the housing (9, 9') located at the end of the housing (9, 9') opposite to the hub (4, 4'), alternatively the rotation of the slider (10", 10‴) in relation to the housing is blocked by the hub (4", 4‴) having four longitudinal guides (39, 39‴) featured on an elongated central element (6'', 6‴) and inserted into internal guide notches (40, 40‴) of the slider (10'', 10‴).

2. The device according to claim 1, **characterised in that** the hub (4, 4', 4'', 4‴) has a means for connecting the hub (4, 4', 4'', 4‴) to the housing (9, 9', 9'', 9‴) located on its circumference as well as the hub (4, 4', 4'', 4‴) has an axial central member (6, 6', 6'', 6‴), in which the second end (3, 3', 3'', 3‴) of the needle (1, 1', 1", 1‴) is placed, and a retaining element for the second end of the longitudinal resilient means (18, 18', 18'', 18‴) surrounding the central member (6, 6', 6", 6‴).

3. The device according to claim 2, **characterised in that** the longitudinal resilient means is a compression helical spring (18, 18', 18'', 18‴) coaxial in relation to the needle (1, 1', 1", 1‴) and surrounding the central member (6, 6', 6'', 6‴).

4. The device according to claim 2 or 3, **characterised in that** the start bay (22, 22", 22‴) of the linear notch (21, 21", 21‴) is located at its first end and is offset against the axis of the linear notch (21, 21", 21‴).

5. The device according to claim 4, **characterised in that** the blocking bay (23, 23", 23‴) of the linear notch (21, 21", 21‴) is in the immediate vicinity of the start bay (22, 22'', 22‴), with the two bays (22, 22'', 22‴, 23, 23'', 23‴) and the linear notch (21, 21", 21"') forming a notch in the side wall of the rotating sleeve (11, 11'', 11‴) with a shape similar to the letter 'Y".

6. The device according to claim 5, **characterised in that** the side wall of the rotary sleeve (11, 11'', 11‴) has two linear notches with bays (21, 21", 21‴, 22, 22", 22‴, 23, 23'', 23‴) located opposite, while the slider (10, 10'', 10‴) has two opposing pins (17, 17", 17‴).

7. The device according to claim 2 or 3, **characterised in that** the blocking bay (23') of the linear notch (21') is located at its first end and is offset against the axis of the linear notch (21'), while the start bay (22') is located between the first and the second end of the linear notch (21').

8. The device according to claim 7, **characterised in that** the side wall of the rotating sleeve (11') has two opposing linear notches (21'), each with two bays (22', 23'), while the slider (10') contains two opposite pins (17').

9. The device according to claim 1, **characterised in that** the slider (10, 10') has two opposing longitudinal guides (13, 13') and the housing (9, 9') has two opposite guide notches (14, 14') corresponding to the longitudinal guides (13, 13') of the slider (10, 10').

10. The device according to claim 1, **characterised in that** the central member (6", 6‴) has four longitudinal guides (39, 39‴) spaced at regular intervals from each other while the slider (10", 10‴) has four guide notches (40, 40‴) corresponding to the longitudinal guides (39, 39‴) of the central member (6", 6‴).

11. The device according to one of the claims 1 to 10, **characterised in that** it contains indicator means (25, 25', 25'', 26, 26', 26'', 26‴, 44) showing the slider (10, 10', 10'', 10‴) is blocked in the position most extended out of the housing (9, 9', 9", 9‴).

12. The device according to claim 11, **characterised in that** the indicator means (25, 25', 25", 26, 26', 26'', 26‴, 44) showing the slider (10, 10', 10'', 10‴) is blocked in the most extended position constitute at least one window (26, 26', 26", 26‴) in the side wall of the housing (9, 9', 9'', 9‴) and at least one mark (25, 25', 25'', 44) on the outer surface of the rotating sleeve (11, 11', 11'', 11‴).

13. The device according to one of the claims 1 to 12, **characterised in that** the hollow of the slider (10, 10') features a narrower section (30, 30') supporting the needle (1, 1').

14. The device according to one of the claims 1 to 13, **characterised in that** the slider (10, 10', 10'', 10‴) is made of transparent plastic.

15. The device according to one of the claims 1 to 14, **characterised in that** the second end (3"') of the needle (1‴) protrudes from the hub (4"') on the opposite side to the first end (2‴) of the needle (1‴).

16. The device according to claim 15, **characterized in that** it includes a stabiliser (42) of the second end (3 "') of the needle (1' "), surrounding the needle (1''') and axially embedded in the hub (4"').

## Patentansprüche

1. Sicherheitsnadelvorrichtung für medizinische Instrumente, umfassend:
- eine längliche Nadel (1, 1', 1", 1"') mit einem achsialen Kanal, deren erstes Ende (2, 2', 2", 2"') zum Einführen in den Körper eines Patienten bestimmt und in einer Nabe (4, 4', 4", 4"') im Bereich ihres (1, 1', 1", 1‴) zweiten Endes (3, 3', 3", 3"') eingebettet ist, wobei die Nabe (4, 4', 4", 4"') mit Mitteln zum Verbinden des Kanals der Nadel (1, 1', 1", 1‴) mit einem Gefäß zur Zufuhr einer injizierbaren Substanz oder einem Gefäß zur Aufnahme einer Probe von Patientengewebe versehen ist;
- ein Gehäuse (9, 9', 9", 9‴), das unbeweglich an der Nabe (4, 4', 4", 4"') befestigt ist und die Nadel (1, 1', 1", 1‴) entlangeines Teils ihrer Länge;
- einen hohlen Schieber (10, 10', 10", 10"'), der die Nadel (1, 1', 1", 1‴) umgibt und sich längs und frei entlang der Achse der Nadel (1, 1', 1", 1‴) bewegt, wobei sein erstes Ende (15, 15', 15", 15"') zur Außenseite des Gehäuses (9, 9', 9", 9‴) gerichtet ist und das zweite Ende (16, 16', 16", 16‴) sich innerhalb des Gehäuses (9, 9', 9", 9‴) bewegt und zur Nabe (4, 4', 4", 4"') gerichtet ist, sowie Mittel, die eine Drehung des Schiebers (13, 13', 14, 14', 39, 39"', 40, 40‴) relativ zum Gehäuse (9, 9', 9", 9"') und ein Herausrutschen des zweiten Endes (16, 16', 16", 16") des Schiebers (10, 10', 10", 10‴) aus dem Gehäuse (9, 9', 9", 9"') verhindern; wobei der Schieber (10, 10', 10", 10"') von einer Ausgangsposition vor der Injektion in eine Endposition nach der Injektion beweglich ist;
- ein längliches elastisches Mittel (18, 18', 18", 18"'), das sich innerhalb des Gehäuses (9, 9', 9", 9‴) befindet und so ausgebildet ist, dass es während der Längsbewegung des Schiebers (10, 10', 10", 10"') innerhalb des Gehäuses (9, 9', 9", 9") allmählich zusammengedrückt wird, wobei sein erstes Ende gegen den Schieber (10, 10', 10", 10") und sein zweites Ende gegen die Nabe (4, 4', 4", 4"') gestützt ist, sowie sich im niedrigsten Spannungszustand befindet, wenn der Schieber (10, 10', 10", 10") mit seinem ersten Ende (15, 15', 15", 15"') am weitesten aus dem Gehäuse (9, 9', 9", 9") ausgefahren ist;
- Mittel zum Blockieren der Bewegung des Schiebers (10, 10', 10", 10") in der am weitesten aus dem Gehäuse (9, 9', 9", 9"') ausgefahrenen Position, nachdem das erste Ende (2, 2', 2", 2‴) der Nadel (1, 1', 1", 1‴) aus dem Körper des Patienten entfernt wurde;
- in dem Gehäuse (9, 9', 9", 9") ein zylindrischer Kanal (20, 20', 20", 20") vorhanden ist, dessen Achse parallel zur Achse der Nadel (1, 1', 1", 1‴) verläuft;
- in dem zylindrischen Kanal (20, 20', 20", 20") dieses Gehäuses (9, 9', 9", 9") eine Drehhülse (11, 11', 11", 11‴) angeordnet ist, die die Nadel (1, 1', 1", 1‴) umgibt und so ausgebildet ist, dass sie sich bei einer Längsbewegung des Schiebers (10, 10', 10", 10") um die Achse der Nadel dreht, ein längliches elastisches Mittel (18, 18', 18", 18"') und mindestens das zweite Ende (16, 16', 16", 16") des Schiebers (10, 10', 10", 10‴);
- in der Seitenwand der Drehhülse (11, 11', 11", 11") mindestens eine lineare Kerbe (21, 21', 21", 21") vorhanden ist, deren Achse entlang der Achse der Nadel (1, 1', 1", 1‴) verläuft, die ein erstes Ende und ein zweites Ende sowie eine Anfangsbucht (22, 22', 22", 22"') und eine Blockierbucht (23, 23', 23", 23‴) aufweist, wobei das erste Ende der linearen Kerbe (21, 21', 21", 21") näher am ersten Ende (2, 2', 2", 2"') der Nadel (1, 1', 1", 1‴) liegt als das zweite Ende der linearen Kerbe (21, 21', 21", 21"');
- in der Nähe des zweiten Endes (16, 16', 16", 16") des Schiebers (10, 10', 10", 10") mindestens ein Stift (17, 17', 17", 17"') vorhanden ist, der quer zur Achse der Nadel (1, 1', 1", 1‴) angeordnet und mechanisch mit der linearen Kerbe (21, 21', 21", 21") in der Seitenwand der Drehhülse (11, 11', 11", 11") gekoppelt ist,
- wobei unter dem Druck des länglichen elastischen Mittels (18, 18', 18", 18") der Schieber (10, 10', 10", 10") vollständig ausgefahren ist und die Nadel (1, 1', 1", 1") vollständig abdeckt, während sich der Stift (17, 17', 17", 17‴) in der Anfangsbucht (22, 22', 22", 22"') befindet und unter dem Druck des länglichen elastischen Mittels (18, 18', 18", 18‴) gegen eine Anfangshaltefläche (28, 28', 28", 28‴) gestützt ist;
- wobei, wenn die Nadel (1, 1', 1", 1") eingeführt wird und infolge der eingeleiteten Bewegung des Schiebers (10, 10', 10", 10"), der Stift (17, 17', 17", 17") den Kontakt mit der Anfangshaltefläche (28, 28', 28", 28‴) verliert und die Anfangsbucht (22, 22', 22", 22") verlässt und aufgrund einer schrägen Neigung der ersten Führungsfläche (29, 29', 29", 29") Druck auf eine erste Führungsfläche (29, 29', 29", 29") der Drehhülse (11, 11', 11", 11‴) ausübt, was bewirkt, dass sich die Drehhülse (11, 11', 11", 11") um die Achse der Nadel (1, 1', 1", 1‴) dreht und sich der Stift (17, 17', 17", 17") in die lineare Kerbe (21, 21', 21", 21‴) bewegt;
- wobei, wenn die Nadel (1, 1', 1", 1‴) zurückgezogen wird und der Schieber (10, 10', 10", 10‴) herausgleitet, sich der Stift (17, 17', 17", 17") in der linearen Kerbe (21, 21', 21", 21") bewegt, bis er eine zweite Führungsfläche (31, 31', 31", 31") der Drehhülse (11, 11', 11", 11") erreicht, wobei diese Fläche eine schräge Anordnung aufweist, die eine weitere Drehung der Drehhülse (11, 11', 11", 11") und das Einführen des Stiftes (17, 17', 17", 17") in eine Blockierbucht (23, 23', 23", 23") und seine Anlage an einer ersten Blockierhaltefläche (32, 32', 32", 32") bewirkt, sodass ein anschließendes Niederdrücken des Schiebers (10, 10', 10", 10‴) den Stift (17, 17', 17", 17") veranlasst, gegen eine zweite Blockierhaltefläche (33, 33', 33", 33"') zu blockieren und seine weitere Bewegung hinein zu verhindern,
- die Drehung des Schiebers (10, 10') in Bezug auf das Gehäuse (9, 9') wird dadurch verhindert, dass der Schieber (10, 10') zwei lineare Führungen (13, 13') auf seiner Außenfläche aufweist, die in Kombination mit Führungsnuten (14, 14') des Gehäuses (9, 9') wirken, die sich am Ende des Gehäuses (9, 9') gegenüber der Nabe (4, 4') befinden, alternativ wird die Drehung des Schiebers (10", 10‴) in Bezug auf das Gehäuse durch die Nabe (4", 4"') blockiert, die vier Längsführungen (39, 39‴) aufweist, die auf einem länglichen Zentralelement (6", 6‴) ausgebildet und in innere Führungsnuten (40, 40") des Schiebers (10", 10") eingeführt sind.

2. Vorrichtung nach Anspruch 1, **dadurch gekennzeichnet, dass** die Nabe (4, 4', 4", 4"') ein Mittel zum Verbinden der Nabe (4, 4', 4", 4"') mit dem Gehäuse (9, 9', 9", 9‴) aufweist, das sich an ihrem Umfang befindet, sowie die Nabe (4, 4', 4", 4"') ein achsiales Zentralelement (6, 6', 6", 6‴) aufweist, in dem das zweite Ende (3, 3', 3", 3") der Nadel (1, 1', 1", 1‴) platziert ist, und ein Halteelement für das zweite Ende des länglichen elastischen Mittels (18, 18', 18", 18‴), das das Zentralelement (6, 6', 6", 6‴) umgibt.

3. Vorrichtung nach Anspruch 2, **dadurch gekennzeichnet, dass** das längliche elastische Mittel eine schraubenförmige Druckfeder (18, 18', 18", 18‴) ist, die koaxial zur Nadel (1, 1', 1", 1"') verläuft und das Zentralelement (6, 6', 6", 6"') umgibt.

4. Vorrichtung nach Anspruch 2 oder 3, **dadurch gekennzeichnet, dass** die Anfangsbucht (22, 22", 22"') der linearen Kerbe (21, 21", 21‴) an deren erstem Ende angeordnet und gegen die Achse der linearen Kerbe (21, 21", 21‴) versetzt ist.

5. Vorrichtung nach Anspruch 4, **dadurch gekennzeichnet, dass** die Blockierbucht (23, 23", 23‴) der linearen Kerbe (21, 21", 21"') in unmittelbarer Nähe der Anfangsbucht (22, 22", 22") liegt, wobei die beiden Buchten (22, 22", 22"', 23, 23", 23"') und die lineare Kerbe (21, 21", 21"') eine Kerbe in der Seitenwand der Drehhülse (11, 11", 11‴) mit einer dem Buchstaben 'Y' ähnlichen Form bilden.

6. Vorrichtung nach Anspruch 5, **dadurch gekennzeichnet, dass** die Seitenwand der Drehhülse (11, 11", 11"') zwei gegenüberliegend angeordnete lineare Kerben mit Buchten (21, 21", 21", 22, 22", 22"', 23, 23", 23"') aufweist, während der Schieber (10, 10", 10‴) zwei gegenüberliegende Stifte (17, 17", 17‴) aufweist.

7. Vorrichtung nach Anspruch 2 oder 3, **dadurch gekennzeichnet, dass** die Blockierbucht (23') der linearen Kerbe (21') an deren erstem Ende angeordnet und gegen die Achse der linearen Kerbe (21') versetzt ist, während die Anfangsbucht (22') zwischen dem ersten und dem zweiten Ende der linearen Kerbe (21') angeordnet ist.

8. Vorrichtung nach Anspruch 7, **dadurch gekennzeichnet, dass** die Seitenwand der Drehhülse (11') zwei gegenüberliegende lineare Kerben (21') aufweist, jeweils mit zwei Buchten (22', 23'), während der Schieber (10') zwei gegenüberliegende Stifte (17') enthält.

9. Vorrichtung nach Anspruch 1, **dadurch gekennzeichnet, dass** der Schieber (10, 10') zwei gegenüberliegende Längsführungen (13, 13') aufweist und das Gehäuse (9, 9') zwei gegenüberliegende Führungsnuten (14, 14') aufweist, die den Längsführungen (13, 13') des Schiebers (10, 10') entsprechen.

10. Vorrichtung nach Anspruch 1, **dadurch gekennzeichnet, dass** das Zentralelement (6", 6‴) vier Längsführungen (39, 39‴) aufweist, die in regelmäßigen Abständen voneinander angeordnet sind, während der Schieber (10", 10‴) vier Führungsnuten (40, 40") aufweist, die den Längsführungen (39, 39‴) des Zentralelements (6", 6‴) entsprechen.

11. Vorrichtung nach einem der Ansprüche 1 bis 10, **dadurch gekennzeichnet, dass** sie Anzeigemittel (25, 25', 25", 26, 26', 26", 26"', 44) enthält, die anzeigen, dass der Schieber (10, 10', 10", 10‴) in der am weitesten aus dem Gehäuse (9, 9', 9", 9"') ausgefahrenen Position blockiert ist.

12. Vorrichtung nach Anspruch 11, **dadurch gekennzeichnet, dass** die Anzeigemittel (25, 25', 25", 26, 26', 26", 26"', 44), die anzeigen, dass der Schieber (10, 10', 10", 10"') in der am weitesten ausgefahrenen Position blockiert ist, mindestens ein Fenster (26, 26', 26", 26"') in der Seitenwand des Gehäuses (9, 9', 9", 9") und mindestens eine Markierung (25, 25', 25", 44) auf der Außenfläche der Drehhülse (11, 11', 11", 11") bilden.

13. Vorrichtung nach einem der Ansprüche 1 bis 12, **dadurch gekennzeichnet, dass** die Aushöhlung des Schiebers (10, 10') einen engeren Abschnitt (30, 30') aufweist, der die Nadel (1, 1') stützt.

14. Vorrichtung nach einem der Ansprüche 1 bis 13, **dadurch gekennzeichnet, dass** der Schieber (10, 10', 10", 10‴) aus transparentem Kunststoff gefertigt ist.

15. Vorrichtung nach einem der Ansprüche 1 bis 14, **dadurch gekennzeichnet, dass** das zweite Ende (3‴) der Nadel (1"') aus der Nabe (4‴) auf der dem ersten Ende (2"') der Nadel (1‴) gegenüberliegenden Seite vorsteht.

16. Vorrichtung nach Anspruch 15, **dadurch gekennzeichnet, dass** sie einen Stabilisator (42) des zweiten Endes (3‴) der Nadel (1‴) einschließt, der die Nadel (1‴) umgibt und axial in der Nabe (4‴) eingebettet ist.

## Revendications

1. Dispositif d'aiguille de sécurité pour instruments médicaux, comprenant :
- une aiguille allongée (1, 1', 1", 1"') avec un canal axial, avec la première extrémité (2, 2', 2", 2"') destinée à être insérée dans le corps d'un patient et noyée dans un embout (4, 4', 4", 4"') dans la zone de sa (1, 1', 1", 1"') seconde extrémité (3, 3', 3", 3"'), l'embout (4, 4', 4", 4"') étant pourvu de moyens pour raccorder le canal de l'aiguille (1, 1', 1", 1‴) à un récipient pour fournir une substance injectable ou à un récipient pour recevoir un échantillon de tissu du patient ;
- un boîtier (9, 9', 9", 9"') fixé de manière inamovible sur l'embout (4, 4', 4", 4"') et entourant l'aiguille (1, 1', 1", 1"') sur une partie de sa longueur ;
- un coulisseau creux (10, 10', 10", 10‴) entourant l'aiguille (1, 1', 1", 1"') et se déplaçant longitudinalement et librement le long de l'axe de l'aiguille (1, 1', 1", 1‴), avec sa première extrémité (15, 15', 15", 15"') dirigée vers l'extérieur du boîtier (9, 9', 9", 9"') et la seconde extrémité (16, 16', 16", 16"') se déplaçant à l'intérieur du boîtier (9, 9', 9", 9"') et dirigée vers l'embout (4, 4', 4", 4"'), ainsi que des moyens empêchant la rotation du coulisseau (13, 13', 14, 14', 39, 39"', 40, 40"') par rapport au boîtier (9, 9', 9", 9"') et la seconde extrémité (16, 16', 16", 16") dudit coulisseau (10, 10', 10", 10"') de sortir du boîtier (9, 9', 9", 9‴) ; le coulisseau (10, 10', 10", 10"') étant mobiled'une position initiale avant l'injection à une position finale après l'injection ;
- un moyen élastique longitudinal (18, 18', 18", 18"') situé à l'intérieur du boîtier (9, 9', 9", 9"') et configuré pour être progressivement comprimé pendant le mouvement longitudinal du coulisseau (10, 10', 10", 10"') à l'intérieur du boîtier (9, 9', 9', 9"), avec sa première extrémité en appui contre le coulisseau (10, 10', 10", 10") et avec sa seconde extrémité en appui contre l'embout (4, 4', 4", 4"'), ainsi qu'étant au niveau de tension le plus bas lorsque le coulisseau (10, 10', 10", 10") est le plus étendu avec sa première extrémité (15, 15', 15", 15") hors du boîtier (9, 9', 9", 9") ;
- des moyens pour bloquer le mouvement du coulisseau (10, 10', 10", 10") dans la position la plus étendue hors du boîtier (9, 9', 9", 9"') après que la première extrémité (2, 2', 2", 2"') de l'aiguille (1, 1', 1", 1"') est retirée du corps du patient ;
- dans le boîtier (9, 9', 9", 9") il y a un canal cylindrique (20, 20', 20", 20") dont l'axe est parallèle à l'axe de l'aiguille (1, 1', 1", 1‴) ;
- dans le canal cylindrique (20, 20', 20", 20") de ce boîtier (9, 9', 9", 9") sont situés un manchon rotatif (11, 11', 11", 11"') entourant l'aiguille (1, 1', 1", 1"') et configuré pour tourner autour de l'axe de l'aiguille lors du mouvement longitudinal du coulisseau (10, 10', 10", 10"), un moyen élastique longitudinal (18, 18', 18", 18‴) et au moins la seconde extrémité (16, 16', 16", 16") du coulisseau (10, 10', 10", 10"') ;
- dans la paroi latérale du manchon rotatif (11, 11', 11", 11"') il y a aumoins une encoche linéaire (21, 21', 21", 21"') avec son axe le long de l'axe de l'aiguille (1, 1', 1", 1‴), ayant une première extrémité et une seconde extrémité ainsi qu'une baie de départ (22, 22', 22", 22‴) et une baie de blocage (23, 23', 23", 23"'), dans lequel la première extrémité de l'encoche linéaire (21, 21', 21", 21") est plus proche de la première extrémité (2, 2', 2", 2‴) de l'aiguille (1, 1', 1", 1"') que la seconde extrémité de l'encoche linéaire (21, 21', 21", 21‴) ;
- à proximité de la seconde extrémité (16, 16', 16", 16") du coulisseau (10, 10', 10", 10‴) il y a au moins une broche (17, 17', 17", 17‴) située transversalement à l'axe de l'aiguille (1, 1', 1", 1‴) et couplée mécaniquement à l'encoche linéaire (21, 21', 21", 21") dans la paroi latérale du manchon rotatif (11, 11', 11", 11"),
- dans lequel, sous la pression du moyen élastique longitudinal (18, 18', 18", 18"), le coulisseau (10, 10', 10", 10") est complètement étendu et couvre complètement l'aiguille (1, 1', 1", 1"), tandis que la broche (17, 17', 17", 17"') est dans la baie de départ (22, 22', 22", 22"') et sous la pression du moyen élastique longitudinal (18, 18', 18", 18"') est supportée contre une surface de retenue de départ (28, 28', 28", 28‴) ;
- dans lequel, lorsque l'aiguille (1, 1', 1", 1") est insérée et en conséquence du mouvement du coulisseau (10, 10', 10", 10"') initié, la broche (17, 17', 17", 17") perd le contact avec la surface de retenue de départ (28, 28', 28", 28"') et sort de la baie de départ (22, 22', 22", 22") et exerce une pression sur une première surface de guidage (29, 29', 29", 29") du manchon rotatif (11, 11', 11", 11"') en raison d'une pente oblique de la première surface de guidage (29, 29', 29", 29"), amenant le manchon rotatif (11, 11', 11", 11") à tourner autour de l'axe de l'aiguille (1, 1', 1", 1‴) et la broche (17, 17', 17", 17") se déplace dans l'encoche linéaire (21, 21', 21", 21‴) ;
- dans lequel, lorsque l'aiguille (1, 1', 1", 1"') est retirée et que le coulisseau (10, 10', 10", 10"') glisse vers l'extérieur, la broche (17, 17', 17", 17") se déplace dans l'encoche linéaire (21, 21', 21", 21") jusqu'à ce qu'elle atteigne une seconde surface de guidage (31, 31', 31", 31") du manchon rotatif (11, 11', 11", 11"), ladite surface ayant une disposition oblique provoquant une rotation supplémentaire du manchon rotatif (11, 11', 11", 11") et l'insertion de la broche (17, 17', 17", 17") dans une baie de blocage (23, 23', 23", 23") et son placement contre une première surface de retenue de blocage (32, 32', 32", 32"), de sorte qu'une dépression ultérieure du coulisseau (10, 10', 10", 10") amènera la broche (17, 17', 17", 17") à se bloquer contre une seconde surface de retenue de blocage (33, 33', 33", 33"), empêchant son mouvement ultérieur,
- la rotation du coulisseau (10, 10') par rapport au boîtier (9, 9') est empêchée par le coulisseau (10, 10') ayant deux guides linéaires (13, 13') sur sa surface extérieure, travaillant en combinaison avec des encoches de guidage (14, 14') du boîtier (9, 9') situées à l'extrémité du boîtier (9, 9') opposée à l'embout (4, 4'), alternativement la rotation du coulisseau (10", 10‴) par rapport au boîtier est bloquée par l'embout (4", 4"') ayant quatre guides longitudinaux (39, 39‴) présents sur un élément central allongé (6", 6"') et insérés dans des encoches de guidage internes (40, 40") du coulisseau (10", 10"').

2. Dispositif selon la revendication 1, **caractérisé en ce que** l'embout (4, 4', 4", 4"') possède un moyen pour connecter l'embout (4, 4', 4", 4"') au boîtier (9, 9', 9", 9"') situé sur sa circonférence ainsi que l'embout (4, 4', 4", 4"') possède un élément central axial (6, 6', 6", 6‴), dans lequel la seconde extrémité (3, 3', 3", 3") de l'aiguille (1, 1', 1", 1"') est placée, et un élément de retenue pour la seconde extrémité du moyen élastique longitudinal (18, 18', 18", 18‴) entourant l'élément central (6, 6', 6", 6"').

3. Dispositif selon la revendication 2, **caractérisé en ce que** le moyen élastique longitudinal est un ressort hélicoïdal de compression (18, 18', 18", 18"') coaxial par rapport à l'aiguille (1, 1', 1", 1‴) et entourant l'élément central (6, 6', 6", 6"').

4. Dispositif selon la revendication 2 ou 3, **caractérisé en ce que** la baie de départ (22, 22", 22"') de l'encoche linéaire (21, 21", 21"') est située à sa première extrémité et est décalée par rapport à l'axe de l'encoche linéaire (21, 21", 21‴).

5. Dispositif selon la revendication 4, **caractérisé en ce que** la baie de blocage (23, 23", 23‴) de l'encoche linéaire (21, 21", 21"') est à proximité immédiate de la baie de départ (22, 22", 22"'), les deux baies (22, 22", 22‴, 23, 23", 23‴) et l'encoche linéaire (21, 21", 21"') formant une encoche dans la paroi latérale du manchon rotatif (11, 11", 11‴) avec une forme similaire à la lettre "Y".

6. Dispositif selon la revendication 5, **caractérisé en ce que** la paroi latérale du manchon rotatif (11, 11", 11‴) a deux encoches linéaires avec des baies (21, 21", 21‴, 22, 22", 22‴, 23, 23", 23‴) situées en opposition, tandis que le coulisseau (10, 10", 10‴) a deux broches opposées (17, 17", 17"').

7. Dispositif selon la revendication 2 ou 3, **caractérisé en ce que** la baie de blocage (23') de l'encoche linéaire (21') est située à sa première extrémité et est décalée par rapport à l'axe de l'encoche linéaire (21'), tandis que la baie de départ (22') est située entre la première et la seconde extrémité de l'encoche linéaire (21').

8. Dispositif selon la revendication 7, **caractérisé en ce que** la paroi latérale du manchon rotatif (11') a deux encoches linéaires opposées (21'), chacune avec deux baies (22', 23'), tandis que le coulisseau (10') contient deux broches opposées (17').

9. Dispositif selon la revendication 1, **caractérisé en ce que** le coulisseau (10, 10') a deux guides longitudinaux opposés (13, 13') et le boîtier (9, 9') a deux encoches de guidage opposées (14, 14') correspondant aux guides longitudinaux (13, 13') du coulisseau (10, 10').

10. Dispositif selon la revendication 1, **caractérisé en ce que** l'élément central (6", 6"') a quatre guides longitudinaux (39, 39"') espacés à des intervalles réguliers l'un de l'autre tandis que le coulisseau (10", 10"') a quatre encoches de guidage (40, 40") correspondant aux guides longitudinaux (39, 39"') de l'élément central (6", 6"').

11. Dispositif selon l'une des revendications 1 à 10, **caractérisé en ce qu'**il contient des moyens indicateurs (25, 25', 25", 26, 26', 26", 26"', 44) montrant que le coulisseau (10, 10', 10", 10"') est bloqué dans la position la plus étendue hors du boîtier (9, 9', 9", 9"').

12. Dispositif selon la revendication 11, **caractérisé en ce que** les moyens indicateurs (25, 25', 25", 26, 26', 26", 26"', 44) montrant que le coulisseau (10, 10', 10", 10"') est bloqué dans la position la plus étendue constituent au moins une fenêtre (26, 26', 26", 26"') dans la paroi latérale du boîtier (9, 9', 9", 9") et au moins une marque (25, 25', 25", 44) sur la surface extérieure du manchon rotatif (11, 11', 11", 11"').

13. Dispositif selon l'une des revendications 1 à 12, **caractérisé en ce que** le creux du coulisseau (10, 10') présente une section rétrécie (30, 30') supportant l'aiguille (1, 1').

14. Dispositif selon l'une des revendications 1 à 13, **caractérisé en ce que** le coulisseau (10, 10', 10", 10"') est fait de plastique transparent.

15. Dispositif selon l'une des revendications 1 à 14, **caractérisé en ce que** la seconde extrémité (3‴) de l'aiguille (1‴) fait saillie de l'embout (4"') du côté opposé à la première extrémité (2‴) de l'aiguille (1‴).

16. Dispositif selon la revendication 15, **caractérisé en ce qu'**il inclut un stabilisateur (42) de la seconde extrémité (3"') de l'aiguille (1‴), entourant l'aiguille (1"') et noyé axialement dans l'embout (4‴).
